# EUROPEAN PATENT APPLICATION

(11) **EP 2 628 729 A1**
(43) Date of publication of application: **21.08.2013**
(21) Application number: 11832068.8
(22) Date of filing: 07.10.2011
(51) Int. Cl.: C07D 219/02, C07D 401/04, C07D 405/14, C07D 409/14, H01L 51/50

(54) **AROMATIC HETEROCYCLIC DERIVATIVE, AND ORGANIC ELECTROLUMINESCENT ELEMENT COMPRISING SAME**

(30) Priority: 12.10.2010 JP 2010229987
(71) Applicant: Idemitsu Kosan Co., Ltd., Chiyoda-ku Tokyo 100-8321 (JP)
(72) Inventor: KATO, Tomoki, Sodegaura-shi Chiba 299-0293 (JP); NISHIMURA, Kazuki, Sodegaura-shi Chiba 299-0293 (JP)
(74) Representative: Gille Hrabal
(86) International application number: PCT/JP2011/005668
(87) International publication number: WO 2012/049828

(57) **Abstract**

An aromatic heterocyclic derivative represented by the following formula (1), a material for an organic electroluminescence device and an organic electroluminescence device including these:

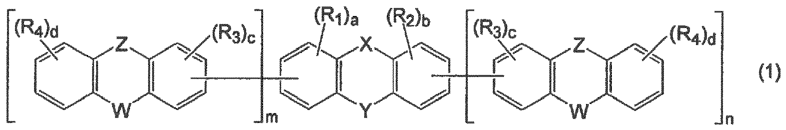

## Description

### TECHNICAL FIELD

The invention relates to an aromatic heterocyclic derivative and an organic electroluminescence device using the same.

### BACKGROUND ART

An organic electroluminescent device (organic EL device) is a promising solid-state emitting type inexpensive and large full-color display device, and has been extensively developed. In general, an organic EL device includes an emitting layer and a pair of opposing electrodes holding the emitting layer therebetween. When an electric field is applied between the electrodes, electrons are injected from a cathode and holes are injected from an anode. The electrons recombine with the holes in the emitting layer to produce an excited state, and energy is emitted as light when the excited state returns to the ground state.
A phosphorous organic EL device utilizing an organic phosphorescent material in the emitting layer of an organic EL device has also been proposed. The phosphorescent organic EL device attains high luminous efficiency by utilizing the singlet and triplet excited states of the organic phosphorescent material. It is considered that when electrons and holes are recombined in the organic EL device, due to the difference in spin multiplicity, a singlet exciton and a triplet exciton are generated at the rate of 1:3. Therefore, if a phosphorescent emitting material is used, it is possible to attain the luminous efficiency which is three to four times as high as that of a device in which only fluorescent material is used. However, in the case of the phosphorescent emitting device, it is required to optimize a triplet energy and the like in addition to optimizing of a singlet energy, HOMO and LUMO, which are necessary for a material for a fluorescent emitting device. Therefore, it is not easy to use the material for a fluorescent emitting device instead. Furthermore, it is difficult for both of fluorescent and phosphorescent emitting devices to attain high efficiency, a long life time and a low voltage at the same time.

Early organic EL devices are insufficient in a driving voltage, luminous efficiency and durability, and various technical improvements have been made for the problems.
The improvements of luminous efficiency and a lifetime of the organic EL device are important subjects which lead to a low power consumption and improvement of durability of display. Therefore, further improvement is required.

To solve the problems, Patent Document 1 discloses that a compound having a structure obtained by connecting two carbazolyl skeletons through a single bond is used as a hole-transporting material instead of N, N'-di(1-naphtyl)-N,N'-diphenyl-(1,1'-biphenyl)-4,4'-diamine(α-NPD), which has been traditionally used, to obtain an organic EL device having high luminance, high luminous efficiency and improved durability.

Patent Document 2 discloses a phenoxazine-based compound as a material having an excellent hole-transporting ability. It describes that bisphenoxazine derivatives can move carriers easily, and exhibit an excellent blue emitting property. However, there is no description which suggests a combination with a phosphorescent emitting layer.
In addition, phenoxazine derivatives have a problem that since they have a small lowest excited triplet energy (T₁), when they are used with phosphorescent materials (in particular, blue phosphorescent materials with a short emission wavelength), they may quench emission of the phosphorescent materials, thereby resulting in a significant decrease in efficiency and insufficient driving durability.
Patent Document 3 discloses an organic EL device having high efficiency, a low driving voltage and high driving durability in which a carrier-transporting material having high chemical stability and a large lowest excited triplet energy (T₁) is used.

However, a material which can be used not only in a fluorescent organic EL device but also in a phosphorescent organic EL device and further improvement of efficiency and a lifetime of the organic EL device are required.

Patent Document 1: JP-A-H08-3547
Patent Document 2: JP-A-2006-199698
Patent Document 3: JP-A-2010-180204

### DISCLOSURE OF THE INVENTION

An object of the invention is to provide a novel compound which can be used not only in a fluorescent organic EL device but also in a phosphorescent organic EL device. Another object of the invention is to provide an organic EL device having high luminous efficiency, a low voltage driving and a long lifetime.

According to the invention, the following aromatic heterocyclic derivative and the like can be provided.
1. An aromatic heterocyclic derivative represented by the following formula (1): 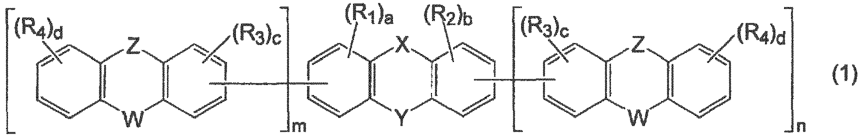 wherein X is CRaRb or SiRaRb,
   Y and W are independently NRc, an oxygen atom or a sulfur atom,
   Z is a single bond, CRaRb or SiRaRb,
   Ra, Rb and Rc are independently a hydrogen atom, a substituted or unsubstituted alkyl group having 1 to 15 carbon atoms, a substituted or unsubstituted cycloalkyl group having 3 to 15 carbon atoms that form a ring (hereinafter referred to as "ring carbon atoms"), a substituted or unsubstituted aralkyl group having 7 to 25 carbon atoms, a substituted or unsubstituted fluoroalkyl group having 1 to 15 carbon atoms, a substituted or unsubstituted aryl group having 6 to 24 ring carbon atoms or a substituted or unsubstituted heteroaryl group having 5 to 24 atoms that form a ring (hereinafter referred to as "ring atoms"),
   R₁, R₂, R₃ and R₄ are independently a substituted or unsubstituted alkyl group having 1 to 15 carbon atoms, a substituted or unsubstituted cycloalkyl group having 3 to 15 ring carbon atoms, a substituted or unsubstituted alkoxy group having 1 to 15 carbon atoms, a substituted or unsubstituted aralkyl group having 7 to 25 carbon atoms, a substituted or unsubstituted fluoroalkyl group having 1 to 15 carbon atoms, a substituted or unsubstituted fluoroalkoxy group having 1 to 15 carbon atoms, a substituted or unsubstituted silyl group, a substituted or unsubstituted aryl group having 6 to 24 ring carbon atoms, a substituted or unsubstituted heteroaryl group having 5 to 24 ring atoms, a substituted or unsubstituted amino group, a substituted or unsubstituted aryloxy group having 6 to 24 ring carbon atoms, a substituted or unsubsituted heteroaryloxy group having 5 to 24 ring atoms, a halogen atom or a cyano group,
   a, b and d are independently an integer of 0 to 4,
   c is an integer of 0 to 3,
   when a to d are independently an integer of 2 or more, two or more of each of R₁s to R₄s may be bonded to each other to form a saturated or unsaturated divalent group,
   m and n are independently an integer of 0 to 4, and
   the substituent of each of the "substituted or unsubstituted" group is a group selected from the group consisting of an alkyl group having 1 to 15 carbon atoms, a cycloalkyl group having 3 to 15 ring carbon atoms, an alkoxy group having 1 to 15 carbon atoms, an aralkyl group having 7 to 25 carbon atoms, a fluoroalkyl group having 1 to 15 carbon atoms, a fluoroalkoxy group having 1 to 15 carbon atoms, a silyl group, an alkylsilyl group having 3 to 30 carbon atoms, an arylsilyl group having 8 to 30 carbon atoms, an aryl group having 6 to 24 ring carbon atoms, a heteroaryl group having 5 to 24 ring atoms, an aryloxy group having 6 to 24 ring carbon atoms, a heteroaryloxy group having 5 to 24 ring atoms, a halogen atom and a cyano group,
   provided that the substituent is not a 9,10-dihydroacridinyl group.
2. The aromatic heterocyclic derivative according to 1 represented by the following formula (2): 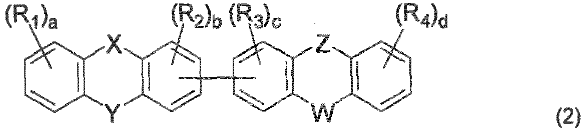 wherein X is CRaRb or SiRaRb,
   Y and W are independently NRc, an oxygen atom or a sulfur atom,
   Z is a single bond, CRaRb or SiRaRb,
   Ra, Rb and Rc are independently a hydrogen atom, a substituted or unsubstituted alkyl group having 1 to 15 carbon atoms, a substituted or unsubstituted cycloalkyl group having 3 to 15 ring carbon atoms, a substituted or unsubstituted aralkyl group having 7 to 25 carbon atoms, a substituted or unsubstituted fluoroalkyl group having 1 to 15 carbon atoms, a substituted or unsubstituted aryl group having 6 to 24 ring carbon atoms or a substituted or unsubstituted heteroaryl group having 5 to 24 ring atoms,
   R₁, R₂, R₃ and R₄ are independently a substituted or unsubstituted alkyl group having 1 to 15 carbon atoms, a substituted or unsubstituted cycloalkyl group having 3 to 15 ring carbon atoms, a substituted or unsubstituted alkoxy group having 1 to 15 carbon atoms, a substituted or unsubstituted aralkyl group having 7 to 25 carbon atoms, a substituted or unsubstituted fluoroalkyl group having 1 to 15 carbon atoms, a substituted or unsubstituted fluoroalkoxy group having 1 to 15 carbon atoms, a substituted or unsubstituted silyl group, a substituted or unsubstituted aryl group having 6 to 24 ring carbon atoms, a substituted or unsubstituted heteroaryl group having 5 to 24 ring atoms, a substituted or unsubstituted amino group, a substituted or unsubstituted aryloxy group having 6 to 24 ring carbon atoms, a substituted or unsubsituted heteroaryloxy group having 5 to 24 ring atoms, a halogen atom or a cyano group,
   a and d are independently an integer of 0 to 4,
   b and c are an integer of 0 to 3,
   when a to d are independently an integer of 2 or more, two or more of each of R₁s to R₄s may be bonded to each other to form a saturated or unsaturated divalent group, and
   the substituent of each of the "substituted or unsubstituted" group is a group selected from the group consisting of an alkyl group having 1 to 15 carbon atoms, a cycloalkyl group having 3 to 15 ring carbon atoms, an alkoxy group having 1 to 15 carbon atoms, an aralkyl group having 7 to 25 carbon atoms, a fluoroalkyl group having 1 to 15 carbon atoms, a fluoroalkoxy group having 1 to 15 carbon atoms, a silyl group, an alkylsilyl group having 3 to 30 carbon atoms, an arylsilyl group having 8 to 30 carbon atoms, an aryl group having 6 to 24 ring carbon atoms, a heteroaryl group having 5 to 24 ring atoms, an aryloxy group having 6 to 24 ring carbon atoms, a heteroaryloxy group having 5 to 24 ring atoms, a halogen atom and a cyano group,
   provided that the substituent is not a 9,10-dihydroacridinyl group.
3. The aromatic heterocyclic derivative according to 2 represented by the following formula (3): 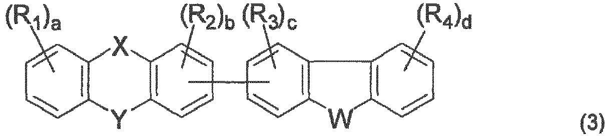 wherein X is CRaRb or SiRaRb,
   Y and W are independently NRc, an oxygen atom or a sulfur atom,
   Ra, Rb and Rc are independently a hydrogen atom, a substituted or unsubstituted alkyl group having 1 to 15 carbon atoms, a substituted or unsubstituted cycloalkyl group having 3 to 15 ring carbon atoms, a substituted or unsubstituted aralkyl group having 7 to 25 carbon atoms, a substituted or unsubstituted fluoroalkyl group having 1 to 15 carbon atoms, a substituted or unsubstituted aryl group having 6 to 24 ring carbon atoms or a substituted or unsubstituted heteroaryl group having 5 to 24 ring atoms,
   R₁, R₂, R₃ and R₄ are independently a substituted or unsubstituted alkyl group having 1 to 15 carbon atoms, a substituted or unsubstituted cycloalkyl group having 3 to 15 ring carbon atoms, a substituted or unsubstituted alkoxy group having 1 to 15 carbon atoms, a substituted or unsubstituted aralkyl group having 7 to 25 carbon atoms, a substituted or unsubstituted fluoroalkyl group having 1 to 15 carbon atoms, a substituted or unsubstituted fluoroalkoxy group having 1 to 15 carbon atoms, a substituted or unsubstituted silyl group, a substituted or unsubstituted aryl group having 6 to 24 ring carbon atoms, a substituted or unsubstituted heteroaryl group having 5 to 24 ring atoms, a substituted or unsubstituted amino group, a substituted or unsubstituted aryloxy group having 6 to 24 ring carbon atoms, a substituted or unsubsituted heteroaryloxy group having 5 to 24 ring atoms, a halogen atom or a cyano group,
   a and d are independently an integer of 0 to 4,
   b and c are an integer of 0 to 3,
   when a to d are independently an integer of 2 or more, two or more of each of R₁s to R₄s may be bonded to each other to form a saturated or unsaturated divalent group, and
   the substituent of each of the "substituted or unsubstituted" group is a group selected from the group consisting of an alkyl group having 1 to 15 carbon atoms, a cycloalkyl group having 3 to 15 ring carbon atoms, an alkoxy group having 1 to 15 carbon atoms, an aralkyl group having 7 to 25 carbon atoms, a fluoroalkyl group having 1 to 15 carbon atoms, a fluoroalkoxy group having 1 to 15 carbon atoms, a silyl group, an alkylsilyl group having 3 to 30 carbon atoms, an arylsilyl group having 8 to 30 carbon atoms, an aryl group having 6 to 24 ring carbon atoms, a heteroaryl group having 5 to 24 ring atoms, an aryloxy group having 6 to 24 ring carbon atoms, a heteroaryloxy group having 5 to 24 ring carbon atoms, a halogen atom and a cyano group,
   provided that the substituent is not a 9,10-dihydroacridinyl group.
4. The aromatic heterocyclic derivative according to 3 represented by the following formula (4): 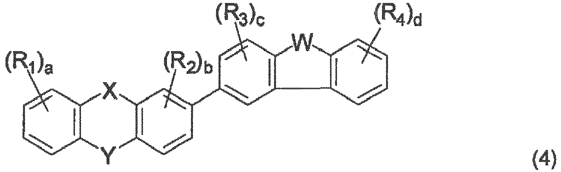 wherein X is CRaRb or SiRaRb,
   Y and W are independently NRc, an oxygen atom or a sulfur atom,
   Ra, Rb and Rc are independently a hydrogen atom, a substituted or unsubstituted alkyl group having 1 to 15 carbon atoms, a substituted or unsubstituted cycloalkyl group having 3 to 15 ring carbon atoms, a substituted or unsubstituted aralkyl group having 7 to 25 carbon atoms, a substituted or unsubstituted fluoroalkyl group having 1 to 15 carbon atoms, a substituted or unsubstituted aryl group having 6 to 24 ring carbon atoms or a substituted or unsubstituted heteroaryl group having 5 to 24 ring atoms,
   R₁, R₂, R₃ and R₄ are independently a substituted or unsubstituted alkyl group having 1 to 15 carbon atoms, a substituted or unsubstituted cycloalkyl group having 3 to 15 ring carbon atoms, a substituted or unsubstituted alkoxy group having 1 to 15 carbon atoms, a substituted or unsubstituted aralkyl group having 7 to 25 carbon atoms, a substituted or unsubstituted fluoroalkyl group having 1 to 15 carbon atoms, a substituted or unsubstituted fluoroalkoxy group having 1 to 15 carbon atoms, a substituted or unsubstituted silyl group, a substituted or unsubstituted aryl group having 6 to 24 ring carbon atoms, a substituted or unsubstituted heteroaryl group having 5 to 24 ring atoms, a substituted or unsubstituted amino group, a substituted or unsubstituted aryloxy group having 6 to 24 ring carbon atoms, a substituted or unsubsituted heteroaryloxy group having 5 to 24 ring atoms, a halogen atom or a cyano group,
   a and d are independently an integer of 0 to 4,
   b and c are an integer of 0 to 3,
   when a to d are independently an integer of 2 or more, two or more of each of R₁s to R₄s may be bonded to each other to form a saturated or unsaturated divalent group, and
   the substituent of each of the "substituted or unsubstituted" group is a group selected from the group consisting of an alkyl group having 1 to 15 carbon atoms, a cycloalkyl group having 3 to 15 ring carbon atoms, an alkoxy group having 1 to 15 carbon atoms, an aralkyl group having 7 to 25 carbon atoms, a fluoroalkyl group having 1 to 15 carbon atoms, a fluoroalkoxy group having 1 to 15 carbon atoms, a silyl group, an alkylsilyl group having 3 to 30 carbon atoms, an arylsilyl group having 8 to 30 carbon atoms, an aryl group having 6 to 24 ring carbon atoms, a heteroaryl group having 5 to 24 ring atoms, an aryloxy group having 6 to 24 ring carbon atoms, a heteroaryloxy group having 5 to 24 ring atoms, a halogen atom or a cyano group,
   provided that the substituent is not a 9,10-dihydroacridinyl group.
5. The aromatic heterocyclic derivative according to any of 1 to 4, wherein X is CRaRb.
6. The aromatic heterocyclic derivative according to any of 1 to 5, wherein Y is NRc.
7. The aromatic heterocyclic derivative according to any of 1 to 6, which is a material for an organic electroluminescence device.
8. The aromatic heterocyclic derivative according to 7, which is a hole-transporting material for an organic electroluminescence device.
9. An organic electroluminescence device comprising:
   a cathode, an anode, and one or more organic thin film layers therebetween,
   wherein one or more layer of the organic thin film layers comprises the aromatic heterocyclic derivative according to any of 1 to 8.
10. The organic electroluminescence device according to 9, wherein the organic thin film layers comprise a hole-transporting layer and/or a hole-injecting layer, and the hole-transporting layer and/or the hole-injecting layer comprises the aromatic heterocyclic derivative.
11. The organic electroluminescence device according to 10, wherein a layer comprising a compound represented by the following formula (10) is in contact with the hole-transporting layer and/or the hole-injecting layer: 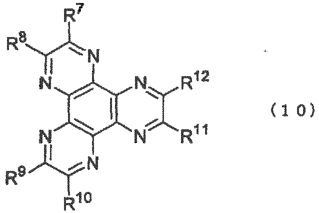 wherein in the formula (10),
   R⁷ to R¹² are independently a cyano group, -CONH₂, a carboxy group or -COOR¹³ wherein R¹³ is an alkyl group having 1 to 20 carbon atoms, or-CO-O-CO- formed by bonding of R⁷and R⁸, R⁹ and R¹⁰, or R¹¹ and R¹² each other.
12. The organic electroluminescence device according to any of 9 to 11, wherein the organic thin film layers comprise an emitting layer, and the emitting layer comprises a phosphorescent material.
13. The organic electroluminescence device according to 12, wherein the phosphorescent material is an ortho-metalated complex of iridium (Ir), osmium (Os) or platinum (Pt).
14. The organic electroluminescence device according to any one of 9 to 13, wherein the organic thin film layers comprise an electron-transporting layer, and the electron-transporting layer comprises the nitrogen-containing heterocyclic derivative represented by any of the following formulas: 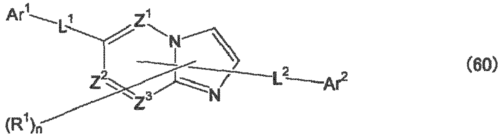 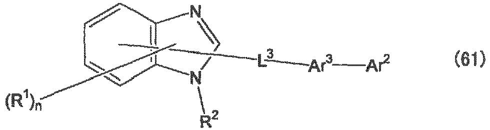 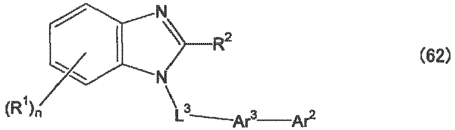 wherein, Z¹, Z² and Z³ are independently a nitrogen atom, a carbon atom or C-H,
   R¹ and R² are independently a substituted or unsubstituted aryl group having 6 to 50 ring carbon atoms, a substituted or unsubstituted heteroaryl group having 3 to 50 ring atoms, an alkyl group having 1 to 20 carbon atoms, an alkyl group having 1 to 20 carbon atoms which is substituted with a halogen atom or an alkoxy group having 1 to 20 carbon atoms,
   n is an integer of 0 to 5,
   when n is an integer of 2 or more, plural R¹s may be the same or different, or adjacent two R¹s may be bonded to each other to form a substituted or unsubstituted aromatic hydrocarbon ring,
   Ar¹ is a substituted or unsubstituted aryl group having 6 to 50 ring carbon atoms or a substituted or unsubstituted heteroaryl group having 3 to 50 ring atoms, and
   Ar² is a halogen atom, an alkyl group having 1 to 20 carbon atoms, an alkyl group having 1 to 20 carbon atoms which is substituted with an halogen atom, an alkoxy group having 1 to 20 carbon atoms, a substituted or unsubstituted aryl group having 6 to 50 ring carbon atoms or a substituted or unsubstituted heteroaryl group having 3 to 50 ring atoms,
   provided that one of Ar¹ and Ar² is a substituted or unsubstituted fused aromatic hydrocarbon ring group having 10 to 50 ring carbon atoms or a substituted or unsubstituted fused aromatic heterocyclic group having 9 to 50 ring atoms,
   Ar³ is a substituted or unsubstituted arylene group having 6 to 50 ring carbon atoms or a substituted or unsubstituted heteroarylene group having 3 to 50 ring atoms, and
   L¹, L² and L³ are independently a single bond, a substituted or unsubstituted arylene group having 6 to 50 ring carbon atoms or a substituted or unsubstituted hetero fused ring group having 9 to 50 ring atoms.

According to the invention, a novel compound which can be used not only in a fluorescent organic EL device but also in a phosphorescent organic EL device can be provided. By using the aromatic heterocyclic derivative of the invention as a hole-transporting material and the like, an organic EL device having high luminous efficiency, low voltage driving and a long lifetime can be obtained.

### BEST MODE FOR CARRYING OUT THE INVENTION

The aromatic heterocyclic derivative of the invention is shown by the following formula (1):

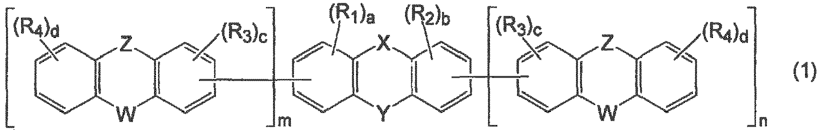

In the formula (1),
X is CRaRb or SiRaRb,
Y and W are independently NRc, an oxygen atom or a sulfur atom,
Z is a single bond, CRaRb or SiRaRb, and
Ra, Rb and Rc are independently a hydrogen atom, a substituted or unsubstituted alkyl group having 1 to 15 carbon atoms, a substituted or unsubstituted cycloalkyl group having 3 to 15 ring carbon atoms, a substituted or unsubstituted aralkyl group having 7 to 25 carbon atoms, a substituted or unsubstituted fluoroalkyl group having 1 to 15 carbon atoms, a substituted or unsubstituted aryl group having 6 to 24 ring carbon atoms or a substituted or unsubstituted heteroaryl group having 5 to 24 ring atoms. It is preferred that Ra, Rb and Rc be a hydrogen atom, a substituted or unsubstituted alkyl group having 1 to 15 carbon atoms, a substituted or unsubstituted aryl group having 6 to 24 ring carbon atoms or a substituted or unsubstituted heteroaryl group having 5 to 24 ring atoms.
R₁, R₂, R₃ and R₄ are independently a substituted or unsubstituted alkyl group having 1 to 15 carbon atoms, a substituted or unsubstituted cycloalkyl group having 3 to 15 ring carbon atoms, a substituted or unsubstituted alkoxy group having 1 to 15 carbon atoms, a substituted or unsubstituted aralkyl group having 7 to 25 carbon atoms, a substituted or unsubstituted fluoroalkyl group having 1 to 15 carbon atoms, a substituted or unsubstituted fluoroalkoxy group having 1 to 15 carbon atoms, a substituted or unsubstituted silyl group, a substituted or unsubstituted aryl group having 6 to 24 ring carbon atoms, a substituted or unsubstituted heteroaryl group having 5 to 24 ring atoms, a substituted or unsubstituted amino group, a substituted or unsubstituted aryloxy group having 6 to 24 ring carbon atoms, a substituted or unsubsituted heteroaryloxy group having 5 to 24 ring atoms, a halogen atom or a cyano group. R₁, R₂, R₃ and R₄ are preferably a substituted or unsubstituted aryl group having 6 to 24 ring carbon atoms or a substituted or unsubstituted heteroaryl group having 5 to 24 ring atoms.
a, b and d are independently an integer of 0 to 4.
c is an integer of 0 to 3.
When a to d are independently an integer of 2 or more, two or more of each of R₁s to R₄s may be bonded to each other to form a saturated or unsaturated divalent group. Preferably, a to d are 0 or 1.
m and n are independently an integer of 0 to 4. Preferably, both of m and n are 0, or at least one of m and n is 1.

As the substituent of the "substituted or unsubstituted" group, a group selected from the group consisting of an alkyl group having 1 to 15 carbon atoms, a cycloalkyl group having 3 to 15 ring carbon atoms, an alkoxy group having 1 to 15 carbon atoms, an aralkyl group having 7 to 25 carbon atoms, a fluoroalkyl group having 1 to 15 carbon atoms, a fluoroalkoxy group having 1 to 15 carbon atoms, a silyl group (-SH₃), an alkylsilyl group having 3 to 30 carbon atoms, an arylsilyl group having 8 to 30 carbon atoms, an aryl group having 6 to 24 ring carbon atoms, a heteroaryl group having 5 to 24 ring atoms, an aryloxy group having 6 to 24 ring carbon atoms, a heteroaryloxy group having 5 to 24 ring atoms, a halogen atom and a cyano group can be given, provided that the substituent is not a 9,10-dihydroacridinyl group represented by the following formula.

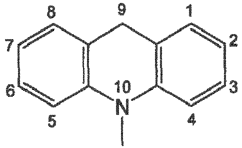

As the substituent, any group can be selected insofar as effects generated by the aromatic heterocyclic derivative represented by the formula (1) appear.
The substituent is preferably an alkyl group having 1 to 15 carbon atoms, an alkylsilyl group having 3 to 30 carbon atoms, an arylsilyl group having 8 to 30 carbon atoms, and an aryl group having 6 to 24 ring carbon atoms, with an alkyl group having 1 to 6 carbon atoms, an alkysilyl group having 3 to 6 carbon atoms, an arylsilyl group having 8 to 14 carbon atoms and an aryl group having 6 to 14 ring carbon atoms being more preferable.

In the specification, the "ring carbon atoms" mean carbon atoms that form a saturated ring, unsaturated ring or aromatic ring. The "ring atoms" mean carbon atoms and hetero atoms that form a saturated ring, unsaturated ring or aromatic ring.
The "unsubstituted" means that a group is substituted with a hydrogen atom and the hydrogen atom of the invention includes light hydrogen, deuterium and tritium.
The aryl group includes a monocyclic aromatic hydrocarbon ring (also referred to as an aromatic hydrocarbon ring) and a fused aromatic hydrocarbon ring. The heteroaryl group includes a monocyclic aromatic heterocyclic ring (also referred to as an aromatic heterocyclic ring) and a fused aromatic heterocyclic ring. Preferably the aryl group or heteroaryl group is one monocyclic ring or fused ring.

The aromatic heterocyclic derivative of the invention represented by the above formula (1) is composed of an aromatic heterocyclic unit represented by the following formula (1-1) and an aromatic heterocyclic unit represented by the following formula (1-2): 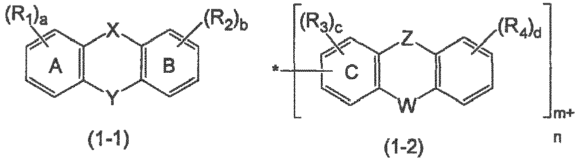 In the above formulas (1-1) and (1-2), X, Y, Z, W, R₁ to R₄, a to d and m and n are the same as those defined in the above formula (1). The symbol * in the unit of the formula (1-2) means a single bond, through which C ring in the unit of the formula (1-2) is connected to at least one of A ring and B ring in the unit of the formula (1-1).

The aromatic heterocyclic derivative of the invention represented by the formula (1) is composed of the unit of the formula (1-1) only, when m=n=0. On the other hand, when at least one of m and n is 1 or more, the derivative has a structure formed by linking the unit of the formula (1-1) and the unit of the formula (1-2).

The aromatic heterocyclic derivative of the invention represented by the formula (1) is characterized in that it has a structure in which two kinds of aromatic heterocyclic units (1-1) and (1-2) are bonded to each other through a benzene ring in the units.
It is considered that when the aromatic heterocyclic rings are bonded to each other through a benzene ring in the units, an unshared electron pair of a heteroatom in the heterocyclic ring lies in the same plane as the benzene ring in the units, thereby to generate an electron-donating effect. Therefore the aromatic heterocyclic ring serves as an electron-donating substituent to increase the electron density of whole molecule.

It is considered that in particular, as shown below, when the para position on A ring or B ring relative to Y in the unit of the formula (1-1) is bonded to the para position on C ring relative to W in the unit of the formula (1-2), the presence of a heteroatom in an aromatic heterocyclic ring results in a significant increase in the electron density of whole molecule to decrease Ip (ionization potential).

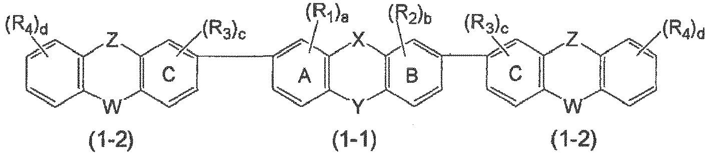

It is considered that by using the aromatic heterocyclic derivative of the invention as a hole-transporting material in contact with an emitting layer, an injection of holes to the emitting layer is promoted so that the driving voltage of an organic EL device can be decreased.
Also, smaller Af (affinity) has an advantage for carrier confinement in the emitting layer to improve the luminous efficiency. In addition, an injection of electrons to the hole-transporting layer is suppressed to obtain an improved lifetime.

Since the aromatic heterocyclic derivative of the invention is composed of aromatic heterocyclic rings only, the singlet energy and the triplet energy thereof are increased as compared to hole-transporting materials having a triphenylamine structure such as NPD, which has been traditionally used. Therefore, use of the derivative as a hole-transporting material in contact with an emitting layer allow effective confinement of carriers and excitons in the emitting layer, thereby to increase luminous efficiency. In particular, since the triplet energy is high, the derivative can be used effectively in the combination with a phosphorescent emitting layer.
Furthermore, the aromatic heterocyclic derivative of the invention can be used as a phosphorescent host.
Also, since Af (affinity) is reduced due to a high singlet energy, an electron resistance is increased, whereby the lifetime of an organic EL device can be improved.
Meanwhile, in the specification, the "triplet energy" means the difference between the energy at lowest triplet excited state and the energy at ground state. The "singlet energy" (also referred to as energy gap) means the difference between the energy at lowest singlet excited state and the energy at ground state.

The aromatic heterocyclic derivative of the invention is preferably represented by the following formula (2): 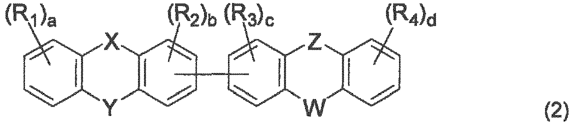 In the formula (2), X, Y, Z, W and R₁ to R₄ are the same as those defined in the above formula (1). a and d are independently an integer of 0 to 4, and b and c are an integer of 0 to 3.
The aromatic heterocyclic derivative represented by the formula (2) is a compound represented by the formula (1) wherein m=0 and n=1.

The aromatic heterocyclic derivative of the invention is more preferably represented by the following formula (3):

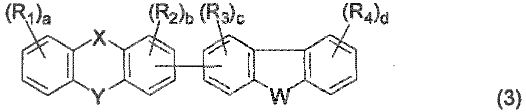

In the formula (3), X, Y, W and R₁ to R₄ are the same as those defined in the above formula (1). a and d are independently an integer of 0 to 4, and b and c are an integer of 0 to 3.
The aromatic heterocyclic derivative represented by the formula (3) is a compound represented by the formula (2) wherein z is a single bond.

The aromatic heterocyclic derivative of the invention is more preferably represented by the following formula (4):

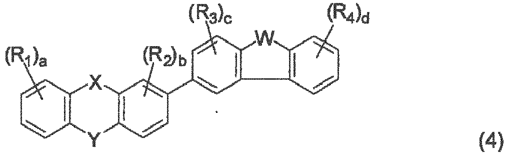

In the formula (4), X, Y, W and R₁ to R₄ are the same as those defined in the above formula (1). a and d are independently an integer of 0 to 4, and b and c are an integer of 0 to 3.
The aromatic heterocyclic derivative represented by the formula (4) is a compound represented by the formula (3) wherein a bonding position between two aromatic heterocyclic rings is specified.

In addition, in the above formulas (1) to (4), it is preferred that X be CRaRb. It is preferred that Y be NRc or an oxygen atom, particularly NRc. It is preferred that W be NRc or an oxygen atom.

The groups in the above-mentioned formulas and the substituents thereof will be described in detail hereinafter.

Examples of the alkyl group include a methyl group, an ethyl group, a propyl group, an isopropyl group, a n-butyl group, a s-butyl group, an isobutyl group, a t-butyl group, a n-pentyl gorup, a n-hexyl group, a n-heptyl group, a n-octyl group and the like.
The alkyl group preferably has 1 to 10 carbon atoms and more preferably 1 to 6 carbon atoms. Particulary, a methyl group, an ethyl group, a propyl group, an isopropyl group, a n-butyl group, a s-butyl group, an isobutyl group, a t-butyl group, a n-pentyl group and a n-hexyl group are preferable.

Examples of the cycloalkyl group include a cyclopropyl group, a cyclobutyl gorup, a cyclopentyl gorup, a cyclohexyl gorup, an adamantyl group, a norbomyl group and the like. The cycloalkyl group has preferably 3 to 10 ring carbon atoms and more preferably 3 to 8 ring carbon atoms.

The aralkyl group is represented by -A-Z. Examples of A include alkylene groups corresponding to the examples of the alkyl group mentioned above. Examples of Z include those exemplified as the aryl group below. The aralkyl group is preferably the one having 7 to 25 carbon atoms, wherein the aryl part has 6 to 24 (preferably 6 to 20, more preferably 6 to 14, particularly preferably 6 to 12) carbon atoms, and the alkylene part has 1 to 15 (preferably 1 to 10, particularly preferably 1 to 6) carbon atoms. For example, a benzyl group, a phenylethyl group or a 2-phenylpropane-2-yl group can be given.

The fluoroalkyl group is an alkyl group substituted with a fluorine atom. Examples thereof include a trifluoromethyl group, a 2,2,2-trifluoroethyl group, a pentafluoroethyl group and the like. The fluoroalkyl group has preferably 1 to 10 carbon atoms, and more preferably 1 to 6 carbon atoms.

Examples of the aryl group include a phenyl group, a 1-naphthyl group, a 2-naphthyl group, a 1-anthryl gorup, a 2-anthryl gorup, a 9-anthryl group, a 1-phenanthryl gorup, a 2-phenanthryl gorup, a 3-phenanthryl gorup, a 4-phenanthryl group, a 9-phenanthryl group, a naphthacenyl group, a pyrenyl group, a chrysenyl group, a benzo[c]phenanthryl gorup, a benzo[g]chrysenyl group, a triphenylenyl group, a 1-fluorenyl gorup, a 2-fluorenyl group, a 3-fluorenyl group, a 4-fluorenyl group, a 9-fluorenyl group, a benzofluorenyl group, a dibenzofluorenyl group, a 2-biphenylyl group, a 3-biphenylyl group, a 4-biphenylyl group, a terphenyl group, a fluoranthenyl group and the like.
The above-mentioned aryl group preferably has 6 to 20 ring carbon atoms and more preferably 6 to 12 ring carbon atoms. A phenyl group, a biphenyl group, a tolyl gorup, a xylyl group and a 1-naphthyl gorup are particulaly preferable among the above-mentioned aryl groups.

Examples of the heteroaryl group include a pyrrolyl group, a pyrazinyl group, a pyridinyl group, an indolyl group, an isoindolyl group, an imidazolyl group, a furyl group, a benzofuranyl group, an isobenzofuranyl group, a 1-dibenzofuranyl group, a 2-dibenzofuranyl group, a 3-dibenzofuranyl group, a 4-dibenzofuranyl group, a 1-dibenzothiophenyl group, a 2-dibenzothiophenyl group, a 3-dibenzothiophenyl group, a 4-dibenzothiophenyl group, a quinolyl group, an isoquinolyl group, a quinoxalinyl group, a 1-carbazolyl group, a 2-carbazolyl group, a 3-carbazolyl group, a 4-carbazolyl group, a 9-carbazolyl group, a phenantridinyl group, an acridinyl group, a phenanthrolinyl group, a phenazinyl group, a phenothiazinyl group, a phenoxazinyl group, an oxazolyl group, an oxadiazolyl group, a furazanyl group, a thienyl group, a benzothiophenyl group and the like.
The above-mentioned heteroaryl group preferably has 5 to 20 ring atoms and more preferably 5 to 14 ring atoms. Among the above-mentioned heteroaryl group, a 1-dibenzofuranyl group, a 2-dibenzofuranyl group, a 3-dibenzofuranyl group, a 4-dibenzofuranyl group, a 1-dibenzothiophenyl group, a 2-dibenzothiophenyl group, a 3-dibenzothiophenyl group, a 4-dibenzothiophenyl group, a 1-carbazolyl group, a 2-carbazolyl group, a 3-carbazolyl group, a 4-carbazolyl group and a 9-carbazolyl group are preferable.

The alkoxy group is represented by -OA. Examples of A include those exemplified as the above-mentioned alkyl group. The alkoxy group is methoxy, ethoxy or the like, for example.
The above-mentioned alkoxy group has preferably 1 to 10 carbon atoms and further preferably 1 to 6 carbon atoms.

The fluoroalkoxy group is represented by -OB. Examples of B include those exemplified as the above-mentioned fluoroalkyl group. The fluoroalkoxy group is trifluoromethoxy, pentafluoroethoxy or the like, for example.
The above-mentioned fluoroalkoxy group has preferably 1 to 10 carbon atoms and further preferably 1 to 6 carbon atoms.

The substituted or unsubstituted silyl group is represented by -SiX¹X²X³ . As examples of X¹, X² and X³, independently, a hydrogen atom and those exemplified as the above-mentioned alkyl group and aryl group can be given. The unsubstituted silyl group is SiH₃. The substituted silyl group includes an alkylsilyl group having 3 to 30 carbon atoms (including mono-, di- and trialkylsilyl groups) and an arylsilyl group having 8 to 30 carbon atoms (including an aryldialkylsilyl group, diarylalkylsilyl group and triarylsilyl group).
Examples of the alkylsilyl group having 3 to 30 (preferably 3 to 20, more preferably 3 to 10) carbon atoms include a trimethylsilyl group, a triethylsilyl group, a t-buthylmethylsilyl group, a vinyldimethylsilyl group, a propyldimethylsilyl group and the like. Examples of the arylsilyl group having 8 to 30 carbon atoms include a triphenylsilyl group, a phenyldimethylsilyl group, a t-butyldiphenylsilyl group, a tritolylsilyl group, a trixylylsilyl group, a trinaphthylsilyl group and the like.

The substituted or unsubstituted amino group is represented by -NHY¹ and -NY²Y³. As examples of Y¹, Y² and Y³, independently, a hydrogen atom and those exemplified as the above-mentioned alkyl group and aryl group can be given.

The aryloxy group is represented by -OZ. As examples of Z, those exemplified as the above-mentioned aryl group can be given.
The aryl group contained in the above-mentioned aryloxy group has preferably 6 to 20, more preferably 6 to 12 ring carbon atoms. Among the above-mentioned aryl groups, a phenyl group, a biphenyl group, a tolyl group, a xylyl group and a 1-naphtyl group are particularly preferable.

The heteroaryloxy group is represented by -OY As examples of Y, those exemplified as the above-mentioned heteroaryl group can be given.
The heteroaryl group contained in the above-mentioned heteroaryloxy group has preferably 5 to 20, more preferably 5 to 14 ring atoms. Further preferable are a 1-dibenzofuranyl group, a 2-dibenzofuranyl group, a 3-dibenzofuranyl group, a 4-dibenzofuranyl group, a 1-dibenzothiophenyl group, a 2-dibenzothiophenyl group, a 3-dibenzothiophenyl group and a 4-dibenzothiophenyl group.

Examples of the aryl group having 6 to 20 ring carbon atoms which is substituted with an aryloxy group include a phenoxyphenyl group, a phenoxynaphthyl group, a naphthoxyphenyl group, a biphenyloxyphenyl group and the like.

Examples of the aryl group having 6 to 20 ring carbon atoms which is substituted with a heteroaryloxy group include a dibenzofuran-1-yloxyphenyl group, a dibenzofuran-2-yloxyphenyl group, a dibenzofuran-3-yloxyphenyl group, a dibenzofuran-4-yloxyphenyl group, a dibenzothiophene-1-yloxyphenyl group, a dibenzothiophene-2-yloxyphenyl group, a dibenzothiophene-3-yloxyphenyl group, a dibenzothiophene-4-yloxyphenyl group, a benzofuran-2-yloxyphenyl group, a benzofuran-3-yloxyphenyl group, a benzothiophene-2-yloxyphenyl group, a benzothiophene-3-yloxyphenyl group, a 1-phenylindole-2-yloxyphenyl group, a 1-phenylindole-3-yloxyphenyl group and the like.

Use of the aryloxy group or the heteroaryloxy group improves the lifetime of an organic EL device, since they have a high oxidative stability compared to alkoxy groups.

Example of the aryl group having 6 to 20 carbon atoms which is substituted with a heteroaryl group include a dibenzofuran-1-ylphenyl group, a dibenzofuran-2-ylphenyl group, a dibenzofuran-3-ylphenyl group, a dibenzofuran-4-ylphenyl group, a dibenzothiophene-1-ylphenyl group, a dibenzothiophene-2-ylphenyl group, a dibenzothiophene-2-ylphenyl group, a dibenzothiophene-4-ylphenyl group, a carbazole-9-ylphenyl group, a benzofuran-2-ylphenyl group, a benzofuran-3-ylphenyl group, a benzothiophene-2-ylphenyl group, a benzothiophene-3-ylphenyl group, a 1-phenylindole-2-ylphenyl group, a 1-phenylindole-3-ylphenyl group, a 5-phenylfuran-2-ylphenyl group, a 5-phenylthiophene-2-ylphenyl group and the like.

As the halogen atom, fluorine, chlorine, bromine, iodine and the like can be given. Fluorine is preferable.

Examples of the aromatic heterocyclic derivative of the invention are given below.

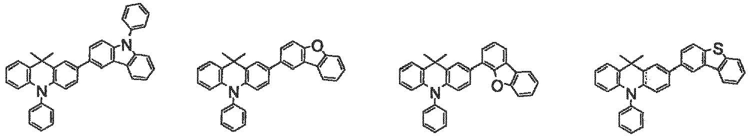

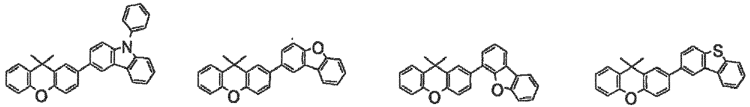

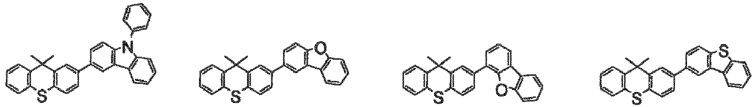

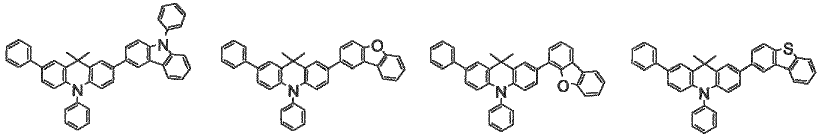

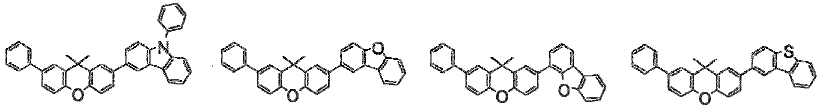

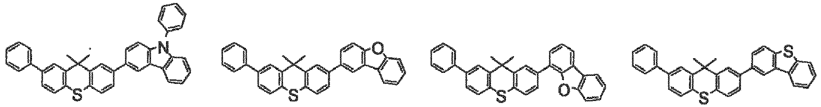

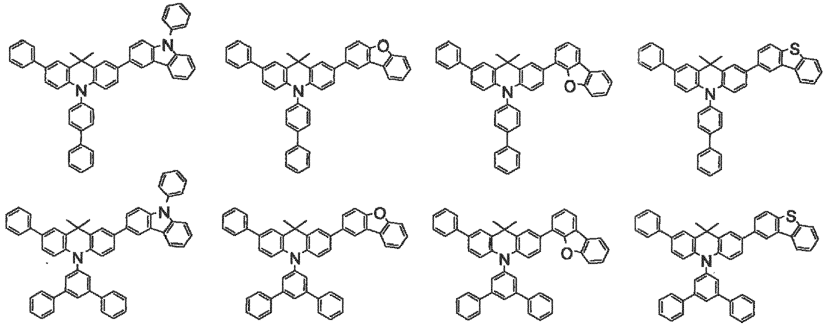

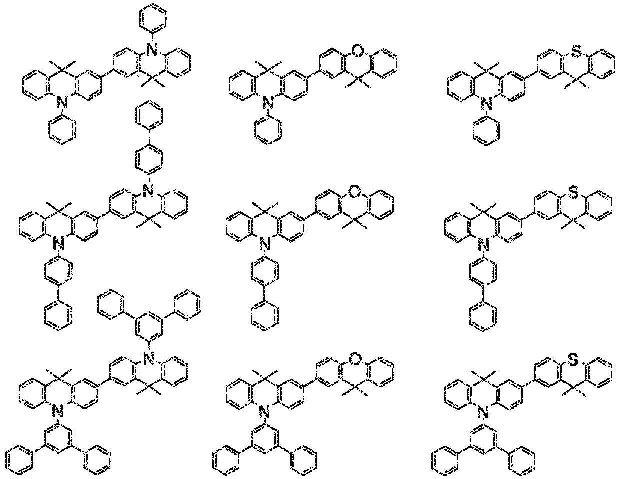

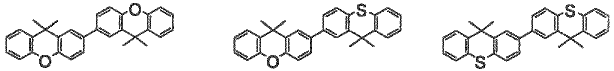

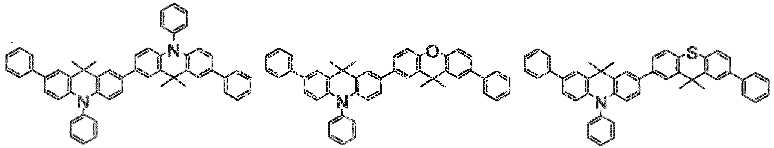

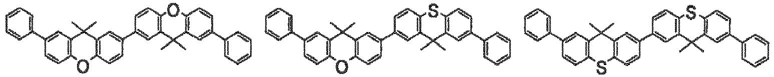

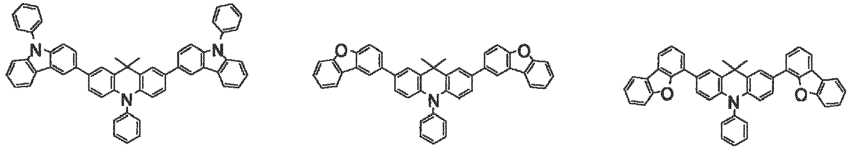

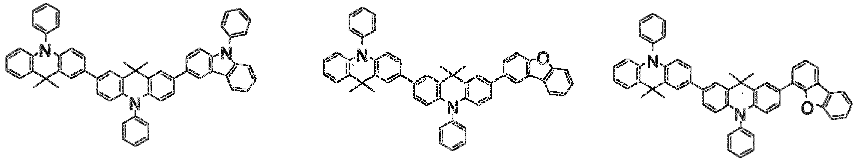

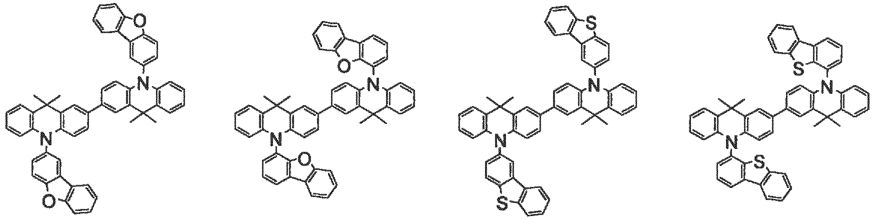

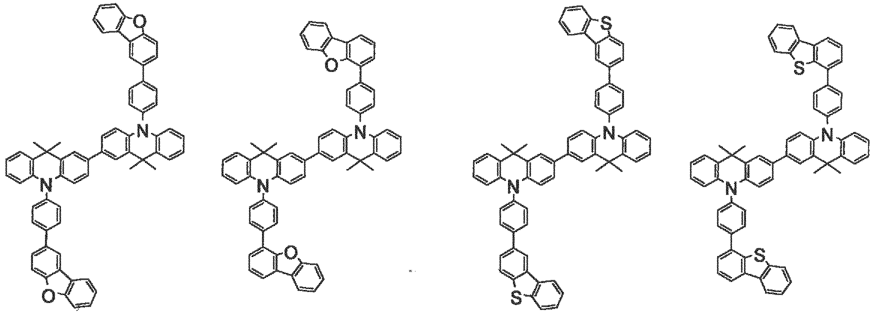

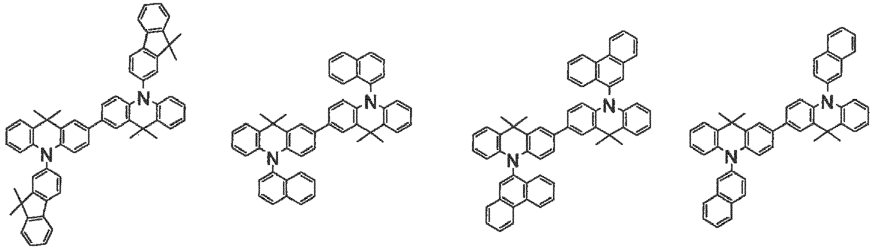

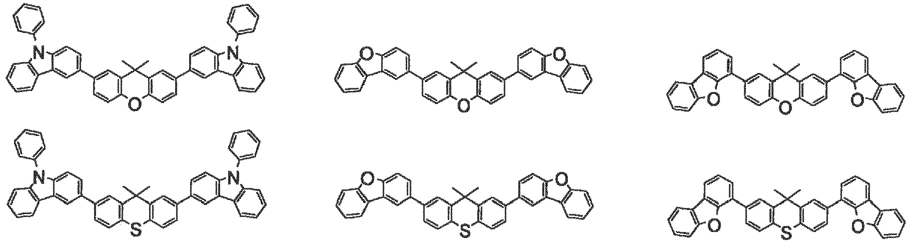

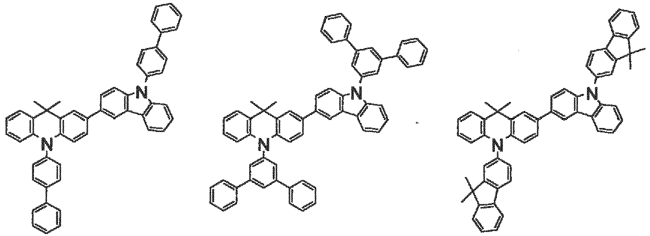

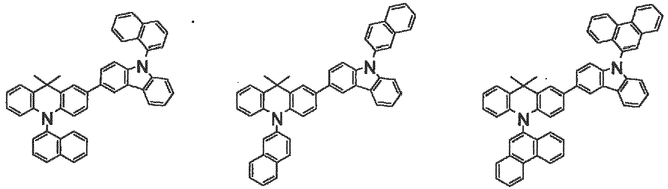

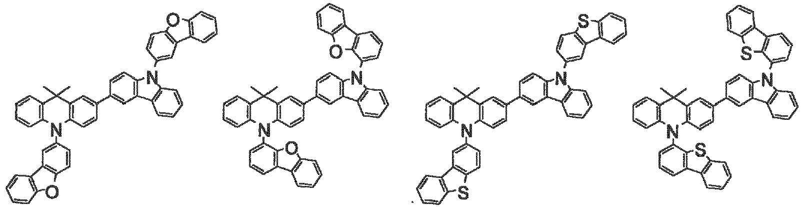

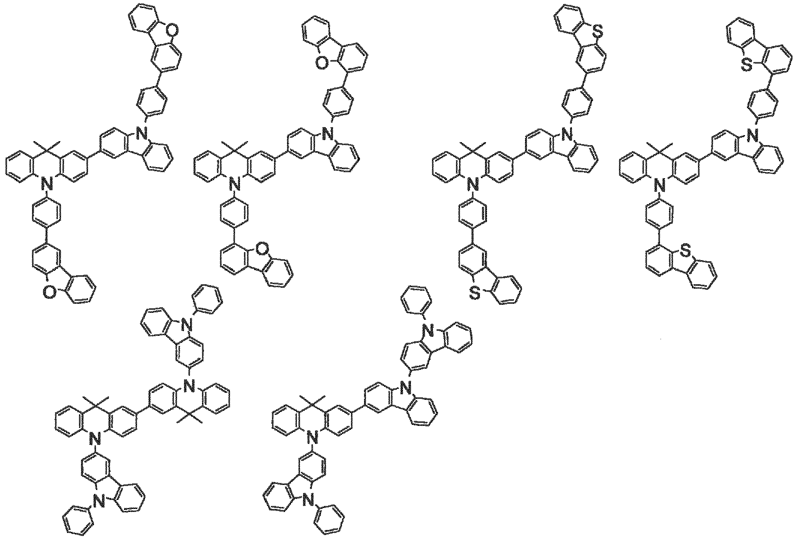

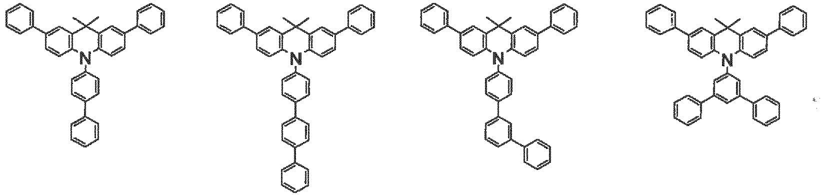

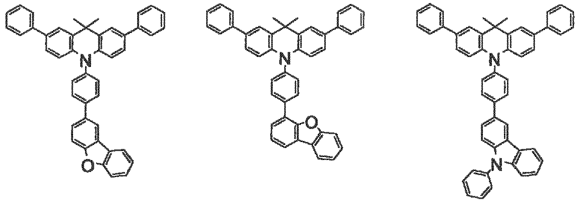

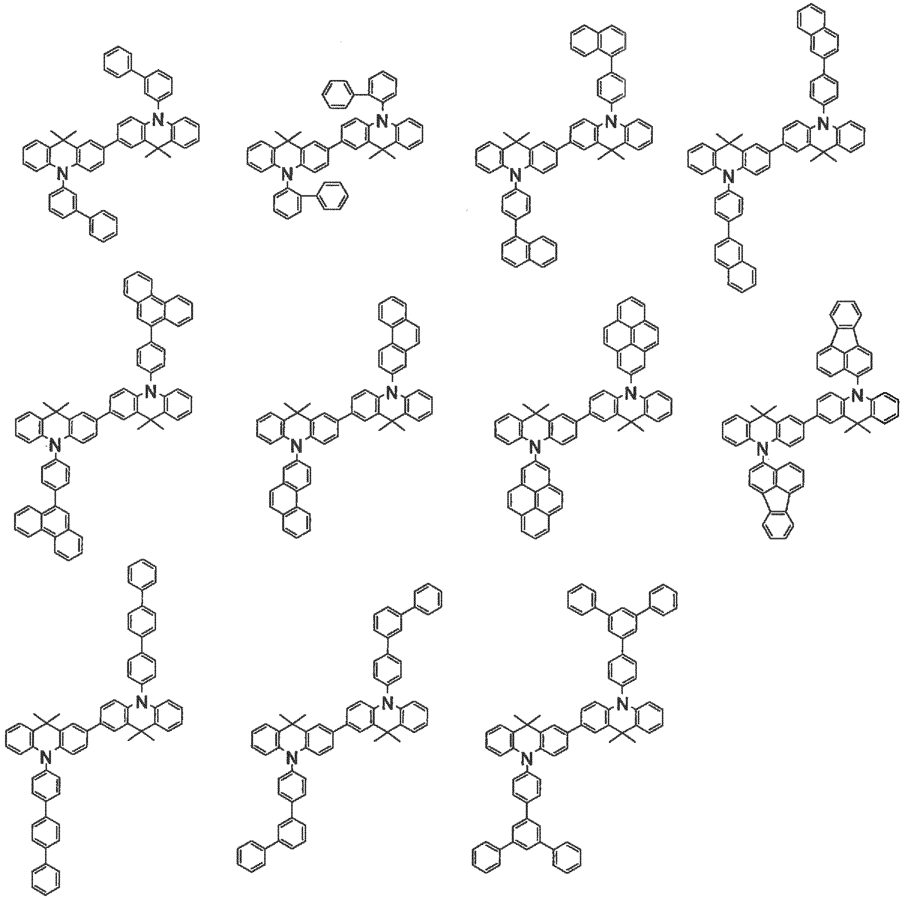

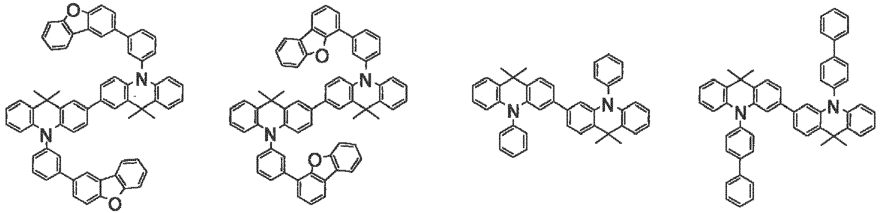

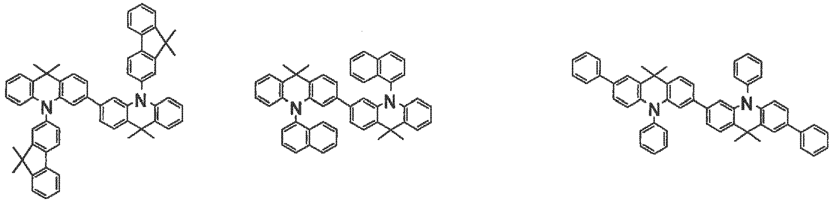

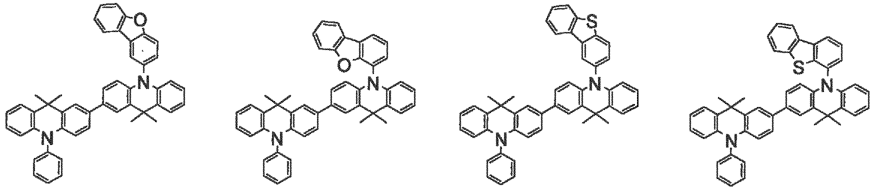

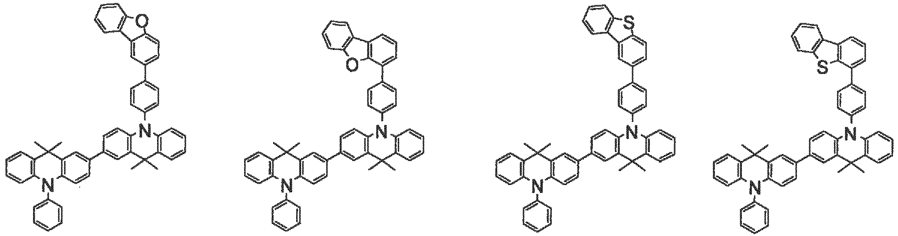

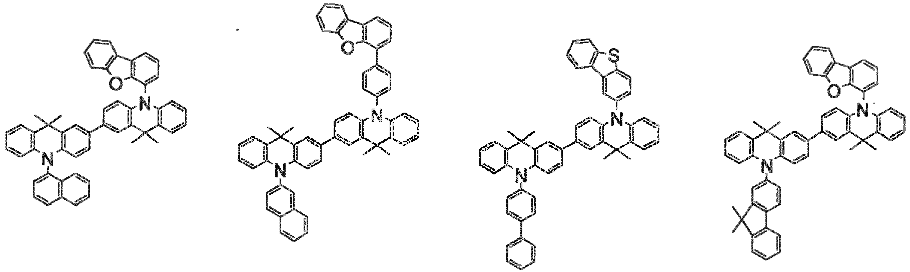

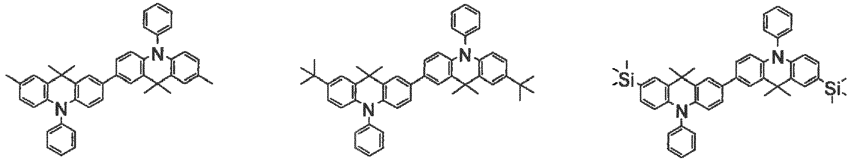

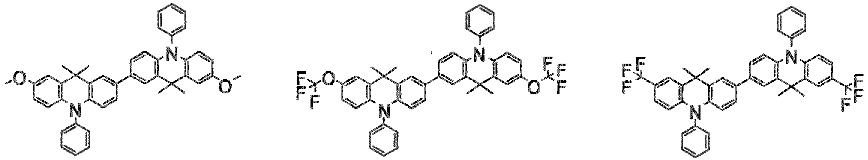

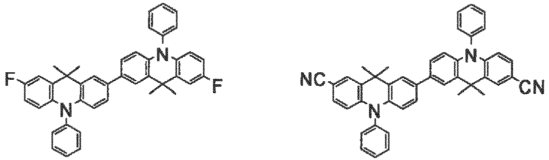

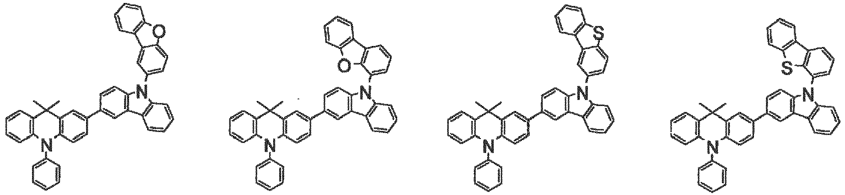

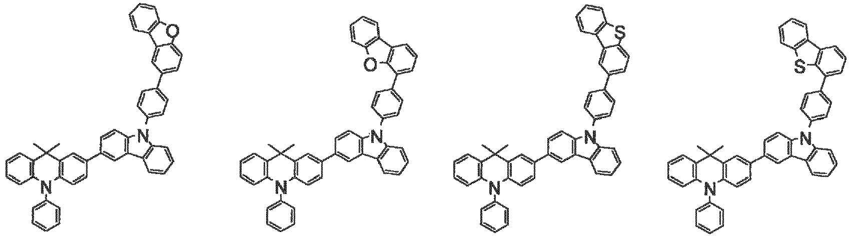

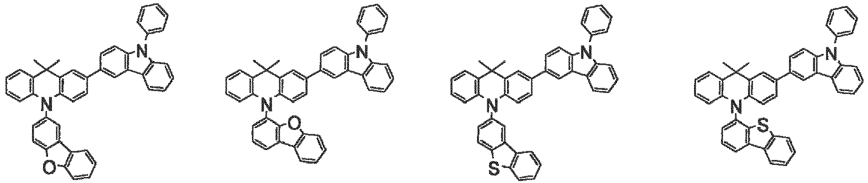

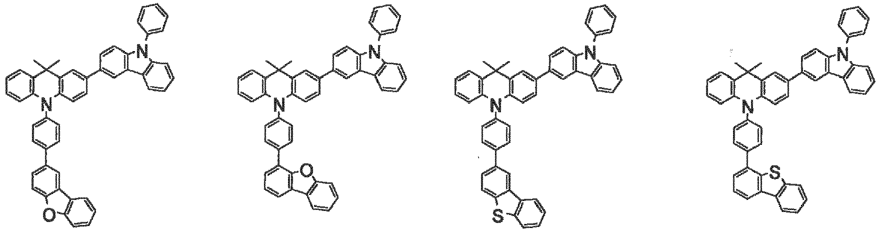

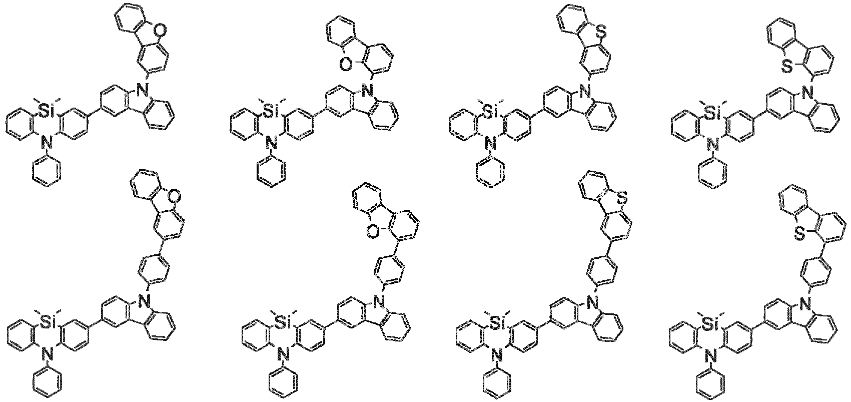

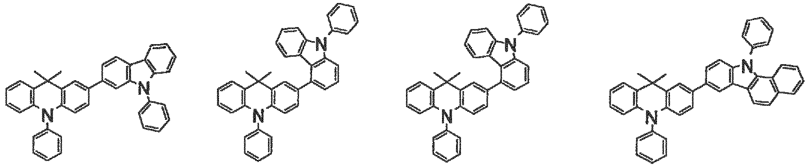

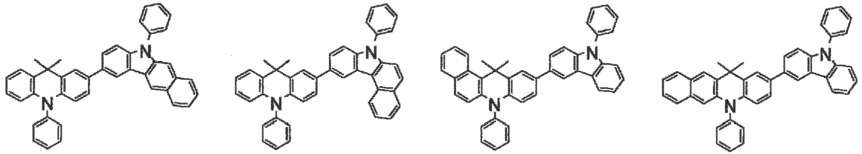

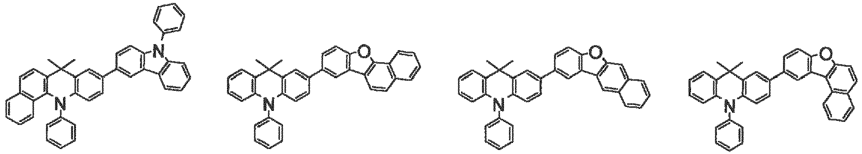

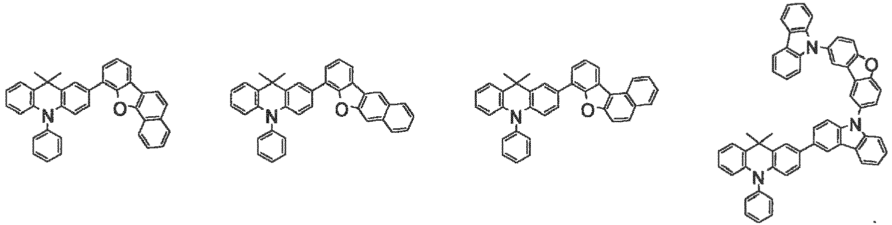

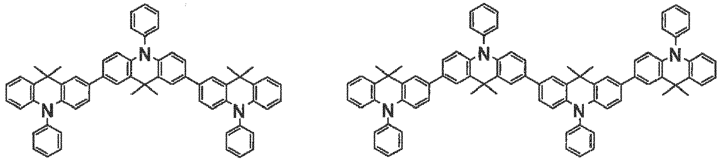

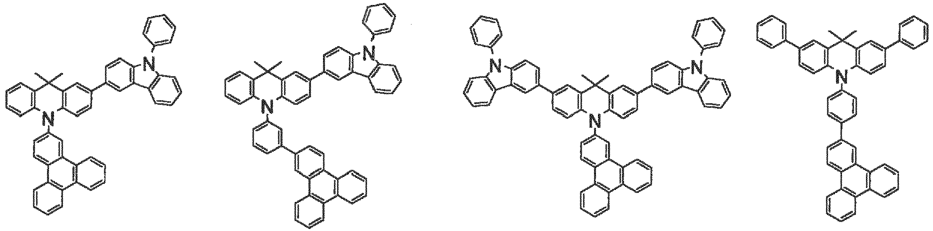

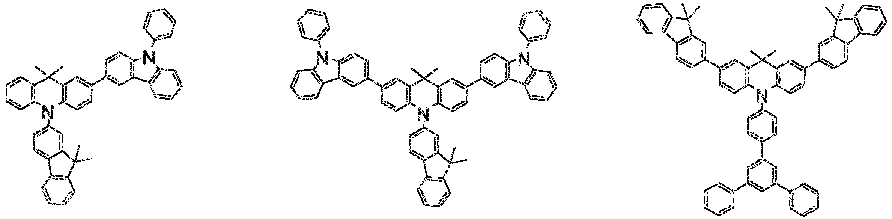

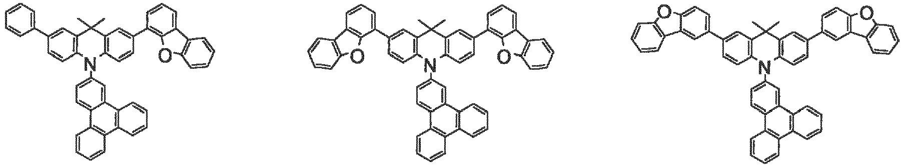

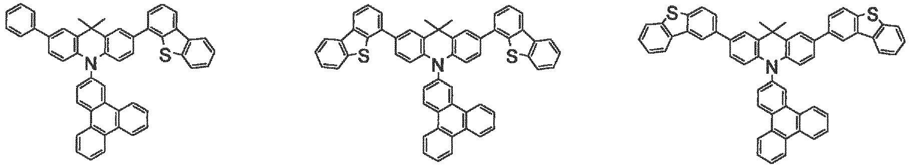

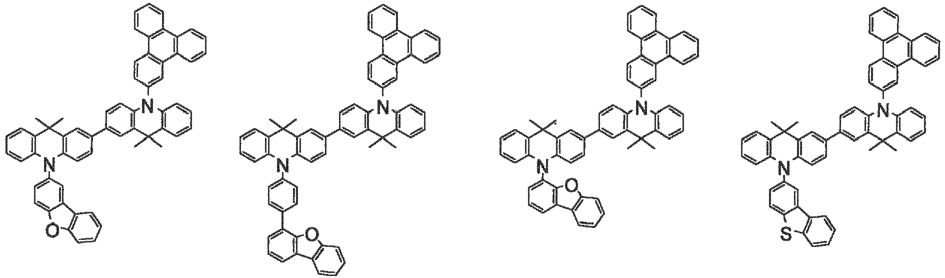

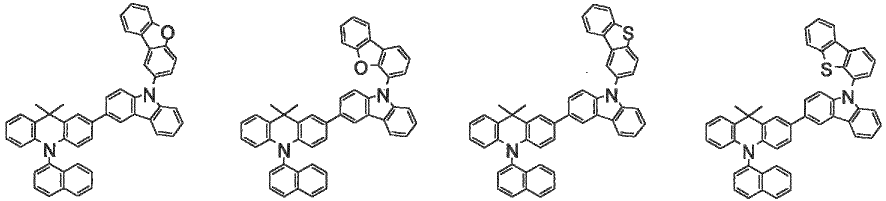

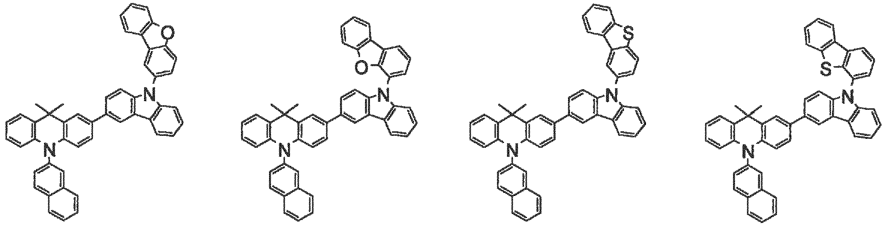

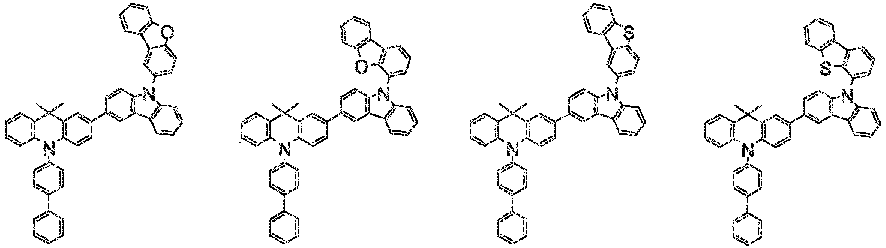

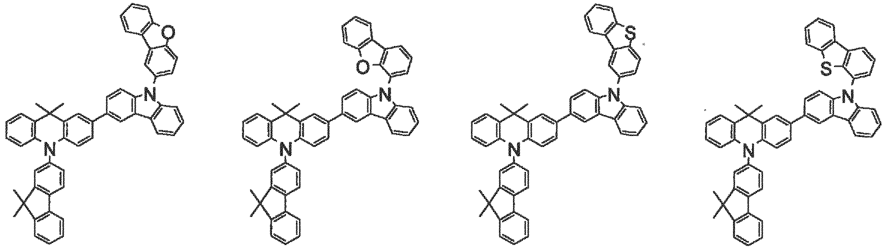

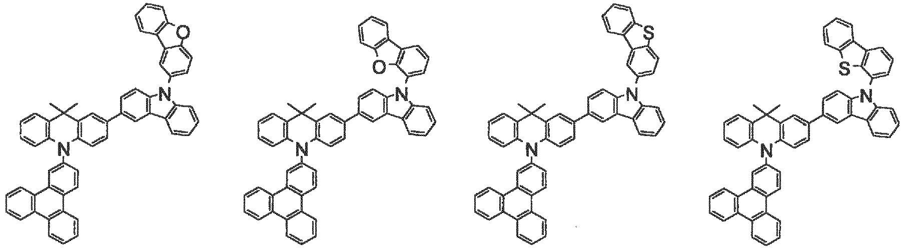

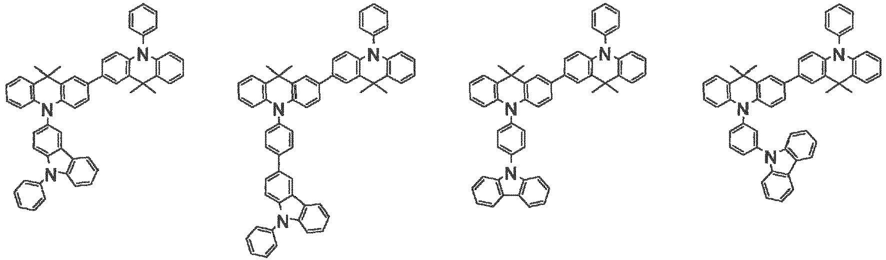

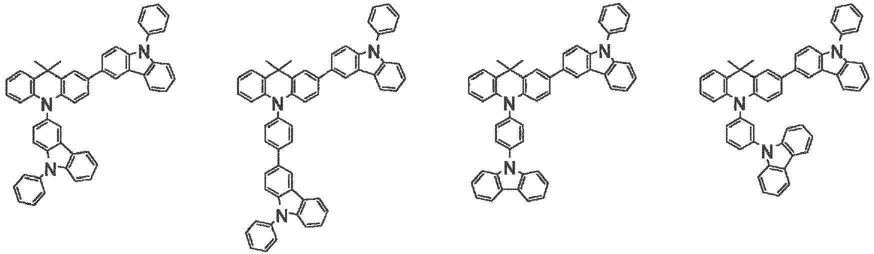

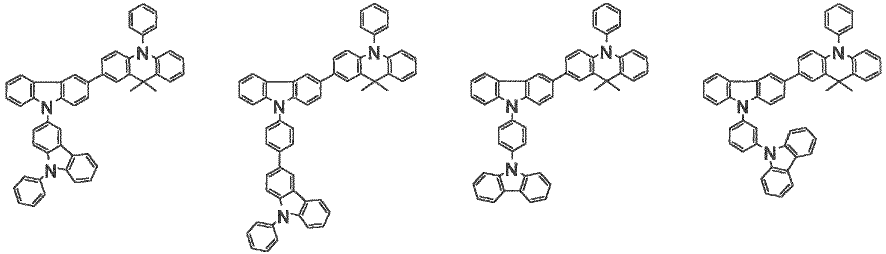

The above aromatic heterocyclic derivatives of the invention can be used as organic EL device materials, preferably hole-transporting materials.
The aromatic heterocyclic derivatives of the invention can be used as materials for a phosphorescent emitting device because of their high triplet energy. By selecting the formulas (1) to (4) appropriately, the derivatives can be used as materials in a plurality of layers such as an emitting layer and a hole-transporting layer in the phosphorescent emitting device.
Furthermore, in the case of the aromatic heterocyclic derivatives represented by the formulas (2) to (4), they can be advantageously used as a hole-transporting material not only in a phosphorescent emitting device but also in a fluorescent emitting device, since they tend to have a small ionization potential (Ip) in addition to high triplet energy.

The organic EL device of the invention includes a cathode, an anode, and one or plural organic thin films including at least an emitting layer between the cathode and the anode, and at least one layer of the organic thin films contains the above-described aromatic heterocyclic derivative. The emitting layer preferably contains a phosphorescent emitting material.
The organic EL device of the invention is not particularly limited so far as the anode, one or more organic thin films and the cathode are stacked in sequence. The organic thin films include an emitting layer and can further include one or more other organic layers. Furthermore, the organic EL device can include one or more inorganic layers.

The organic thin film layers preferably include a hole-transporting layer and/or a hole-injecting layer, and at least one of the hole-transporting layer and the hole-injecting layer contains the above-mentioned aromatic heterocyclic derivative. The hole-transporting layer and/or the hole-injecting layer may contain the aromatic heterocyclic derivative as the major component or may contain the aromatic heterocyclic derivative only.

Furthermore, the emitting layer can contain the aromatic heterocyclic derivative of the invention as a host material.

As the device configuration of the organic EL device, the following first to third embodiments can be given, for example. In these embodiments, the emitting layer may be a multilayer stack of emitting layers. Furthermore, it is preferable that a hole-transporting region be provided between the anode and the emitting layer.

### <First Embodiment>

The organic EL device according to this embodiment has a device configuration in which at least one emitting layer is provided. Specific examples of the configuration are given below.
(1) Anode/emitting layer/electron-injecting and/o- transporting layer/cathode
(2) Anode/hole-injecting and/or -transporting layer/emitting layer/electron-injecting and/or-transporting layer/cathode
(3) Anode/hole-injecting layer/hole-transporting layer/emitting layer/electron-injecting and/or -transporting layer/cathode

In the specification, the "hole-injecting and/or -transporting layer" means one or both of hole-injecting layer and hole-transporting layer, and the "electron-injecting and/or -transporting layer" means one or both of electron-injecting layer and electron-transporting layer. Unless otherwise specified, the emitting layer includes both fluorescent and phosphorescent emitting layers.

### <Second Embodiment>

The organic EL device according to this embodiment has a tandem device configuration in which at least two emitting layers (units having a emitting layer) are provided.
A carrier generation layer (also referred to as CGL) can be provided between the two emitting layers to provide an electron-transporting region for each unit.

Specific examples of the tandem device configuration are given below.
Anode/hole-injecting and/or -transporting layer/fluorescent emitting layer/carrier generation layer/fluorescent emitting layer/electron-injecting and/or -transporting layer/cathode
Anode/hole-injecting and/or -transporting layer/fluorescent emitting layer/electron-injecting and/or -transporting layer/carrier generation layer/fluorescent emitting layer/cathode
Anode/hole-injecting and/or -transporting layer/fluorescent emitting layer/electron-injecting and/or -transporting layer/carrier generation layer/fluorescent emitting layer/blocking layer/cathode
Anode/hole-injecting and/or -transporting layer/phosphorescent emitting layer/carrier generation layer/fluorescent emitting layer/electron-injecting and/or -transporting layer/cathode
Anode/hole-injecting and/or -transporting layer/fluorescent emitting layer/electron-injecting and/or -transporting layer/carrier generation layer/phosphorescent emitting layer/cathode

### <Third Embodiment>

The organic EL device according to this embodiment has a plurality of emitting layers and a carrier-blocking layer between any two of the emitting layers.

As the preferred configuration of the organic EL device according to this embodiment, there can be given the configurations as disclosed in Japanese Patent No. 4134280, US2007/0273270A1 and WO2008/023623A1, and, specifically, the configuration in which an anode, a first emitting layer, a carrier-blocking layer, a second emitting layer and a cathode are sequentially stacked, and an electron-transporting region having a blocking layer for preventing diffusion of triplet excitons is further provided between the second emitting layer and the cathode. The carrier-blocking layer is to adjust a carrier balance of the injected electron and hole in the an emitting layer by providing the energy barriers of HOMO and LUMO levels between the carrier-blocking layer and the adjacent emitting layer thereby to adjust the injection of a carrier into the emitting layer.

The specific examples of such configuration are given below.
Anode/hole-injecting-transporting layer/first emitting layer/carrier-blocking layer/second emitting layer/etectron-injecting·transporting layer/cathode
Anode/hole-injecting·transporting layers/first emitting layer/carrier-blocking layer/second emitting layer/third emitting layer/electron-injecting·transporting layer/cathode

Of the hole-injecting layer and the hole-transporting layer, it is preferable that the layer in contact with the anode include an acceptor material. Furthermore, it is preferable that the layer containing a compound represented by the following formula (10) be in contact with the hole-transporting layer.
Such a configuration realizes a low voltage driving and a high luminous efficiency due to the effects as described in the below-mentioned patent publications.
As the acceptor material, a hexaazatriphenylene derivative as disclosed in Japanese Patent Nos. 3614405 and 3571977 or US Patent No. 4,780,536 can be used. In addition, an inorganic compound such as p-type Si or p-type SiC, an electron accepting inorganic oxide such as a molybdenum oxide, an electron accepting organic compound such as a TCNQ derivative or the like can be preferably used.

As the acceptor material, the compound shown by the following formula (10) or (11) is used preferably.

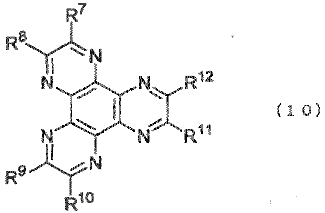

In the above formula (10), R⁷ to R¹² are independently a cyano group, -CONH₂, a carboxyl group or-COOR¹³ (R¹³ is an alkyl group having 1 to 20 carbon atoms), or R⁷ and R⁸, R⁹ and R¹⁰, or R¹¹ and R¹² are bonded to each other to form a group shown by -CO-O-CO-.
Examples of the above alkyl group include a linear, branched or cyclic one, preferably having 1 to 12 carbon atoms, more preferably having 1 to 8 carbon atoms. Specific examples thereof include a methyl group, an ethyl group, a n-propyl group, an isopropyl group, a n-butyl group, a sec-butyl group, a t-butyl group, a n-hexyl group, a n-octyl group, a n-decyl group, a n-hexadecyl group and the like.

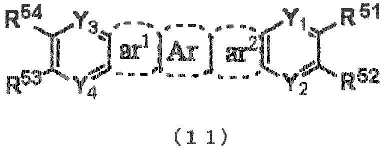

In the above formula (11), Ar is a monocyclic hydrocarbon or fused ring having 6 to 24 ring carbon atoms or a hetero-monocyclic ring or fused ring having 6 to 24 ring atoms. ar¹ and ar² may be the same or different and are shown by the following formula (i) or (ii).

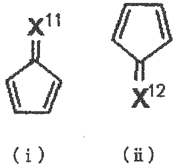

In the formulas, X¹¹ and X¹² may be the same or different and are any of the divalent groups shown by the following formulas (a) to (g).

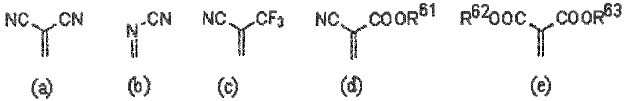

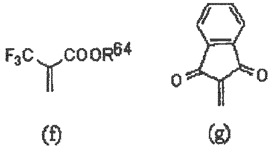

In the formulas, R⁶¹ to R⁶⁴ may be the same or different and are a hydrogen atom, a substituted or unsubstituted fluoroalkyl group having 1 to 20 carbon atoms, a substituted or unsubstituted alkyl group having 1 to 20 carbon atoms, a substituted or unsubstituted aryl group having 6 to 50 carbon atoms, or a substituted or unsubstituted heterocyclic group having 3 to 50 ring atoms. R⁶² and R⁶³ may be bonded to each other to form a ring.

In the formula (11), R⁵¹ to R⁵⁴ may be the same or different and are a hydrogen atom, a substituted or unsubstituted alkyl group having 1 to 20 carbon atoms, a substituted or unsubstituted aryl group having 6 to 50 carbon atoms, a substituted or unsubstituted heterocyclic group having 3 to 50 ring atoms, a halogen atom, a substituted or unsubstituted fluoroalkyl group having 1 to 20 carbon atoms, a substituted or unsubstituted alkoxy group having 1 to 20 carbon atoms, a substituted or unsubstituted fluoroalkoxy group having 1 to 20 carbon atoms, a substituted or unsubstituted aryloxy group having 6 to 50 ring carbon atoms, or a cyano group. Adjacent groups of R⁵¹ to R⁵⁴may be bonded to each other to form a ring. Y¹ to Y⁴ may be the same or different, and are -N=, -CH=, or C(R⁵⁵)=. R⁵⁵ is a substituted or unsubstituted alkyl group having 1 to 20 carbon atoms, a substituted or unsubstituted aryl group having 6 to 50 carbon atoms, a substituted or unsubstituted heterocyclic group having 3 to 50 ring atoms, a halogen atom, a substituted or unsubstituted fluoroalkyl group having 1 to 20 carbon atoms, a substituted or unsubstituted alkoxy group having 1 to 20 carbon atoms, a substituted or unsubstituted fluoroalkoxy group having 1 to 20 carbon atoms, a substituted or unsubstituted aryloxy group having 6 to 50 ring carbon atoms, or a cyano group.

It is preferred that the organic EL device of the invention contain at least one of the carbazolyl compounds represented by the following formula (20) and (21) in at least one layer of the organic thin film layers, preferably in the emitting layer. It is preferable that the emitting layer contain the carbazolyl compound as a host material.

(Cz-)ₐA³ (20)

Cz(-A³)_{b} (21)

In the formulas (20) and (21), Cz is a substituted or unsubstituted arylcarbazolyl group, a substituted or unsubstituted carbazolylaryl group or a substituted or unsubstituted carbazolylalkylene group.
A³ is a group represented by the following formula (A).
a and b are independently an integer of 1 to 3.

(M¹)_{c}-(L⁵)_{d}-(M²)ₑ (A)

In the formula (A), M¹ and M² are independently a substituted or unsubstituted nitrogen-containing aromatic heterocyclic ring or nitrogen-containing fused aromatic heterocyclic ring having 3 to 40 ring atoms, and may be the same or different
L⁵ is a single bond, a substituted or unsubstituted aromatic hydrocarbon ring group or fused aromatic hydrocarbon ring group having 6 to 30 carbon atoms, a substituted or unsubstituted cycloalkylene group having 5 to 30 carbon atoms or a substituted or unsubstituted aromatic heterocyclic ring group or fused aromatic heterocyclic ring group having 2 to 30 carbon atoms.
c is an integer of 0 to 2, d is an integer of 1 to 2 and e is an integer of 0 to 2, provided that c+e is 1 or more.

The compounds represented by the formulas (20) and (21) will be explained in detail below.
Cz is a substituted or unsubstituted arylcarbazolyl group, a substituted or unsubstituted carbazolylaryl group or a substituted or unsubstituted carbazolylalkylene group.

The arylcarbazolyl group is a carbazolyl group having at least one aryl group or heteroaryl group as a substituent, and there is no restriction on the position in the carbazolyl group at which the carbazolyl group is substituted with the aryl group or the heteroaryl group.
Specifically, the following groups can be given, for example. In the following formulas, Ar is an aryl group or a heteroaryl group, and the symbol* indicates the position at which other groups is bonded.

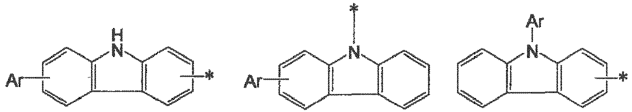

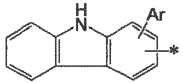

On the other hand, the carbazolylaryl group is an aryl group having at least one carbazolyl group as a substituent, and there is no restriction on the position at which the aryl group is substituted.
Specifically, the following groups can be given, for example. In the following formulas, Ar indicates an aryl group, and the symbol* indicates the position at which other groups bond.

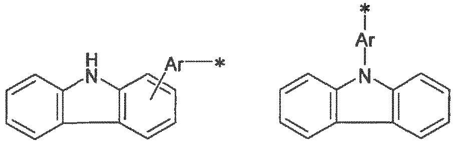

The carbazolylalkylene group is an alkylene group having at least one carbazolyl group as a substituent, and there is no restriction on the position at which the alkylene group is substituted.
Specifically, in the above-mentioned formulas representing the carbazolyaryl group, groups in which Ar is an alkylene group can be given.
The substituted arylcarbazolyl group is the above-mentioned arylcarbazolyl group having at least one substituent regardless of the substitution position. The substituted carbazolylaryl group is the above-mentioned carbazolylaryl group having at least one substituent regardless of the substitution position.

In the formulas (20) and (21), a and b are each an integer of 1 to 3.

The aryl group in the arylcarbazolyl group or the carbazolylaryl group has preferably 6 to 30 carbon atoms. Examples thereof include a phenyl group, a naphthyl group, an anthryl group, a phenanthryl group, a naphthacenyl group, a pyrenyl group, a fluorenyl group, a biphenyl group, a terphenyl group and the like. Among these, a phenyl group, a naphthyl group, a biphenyl group and a terphenyl group are preferable.

Examples of the heteroaryl group in the heteroarylcarbazolyl group include pyridine, pyrimidine, pyrazine, triazine, azirizine, azaindolizine, indolizine, imidazole, indole, isoindole indazole, purine, apteridine, β-carboline, naphthyridine, quinoxaline, terpyridine, bipyridine, acridine, phenanthroline, phenazine, imidazopyridine and the like. In particular, the group formed by the ring of pyridine, terpyridine, pyrimidine, imidazopyridine or triazine is preferable.

The alkylene group in the carbazolylalkylene group preferably has 1 to 10 carbon atoms. Specific examples thereof include a methylene group, an ethylene group, a propylene group, an isopropylene group, a n-butylene group, a s-butylene group, a t-butylene group, an isobutylene group, a n-pentylene group, a n-hexylene group, a n-heptylene group, a n-octylene group, a hydroxymethylene group, a chloromethylene group, an aminomethylene group and the like. Among these, a methylene group, an ethylene group, a propylene group, an isopropylene group, a n-butylene gorup, a t-butylene group and a n-pentylene group are preferable.

In the formulas (20) and (21), A³ is a group represented by the formula (A).
In the formula (A), preferably, M¹ and M² are independently a substituted or unsubstituted nitrogen-containing heterocyclic group having 3 to 40 ring atoms, and may be the same or different.

Examples of the nitrogen-containing heterocyclic group include pyridine, pyrimidine, pyrazine, triazine, azirizine, azaindolizine, indolizine, imidazole, indole, isoindole, indazole, purine, pteridine, β-carboline, naphthyridine, quinoxaline, terpyridine, bipyridine, acridine, phenanthroline, phenazine, imidazopyridine and the like. In particular, a group formed by the ring of pyridine, terpyridine, pyrimidine, imidazopyridine or triazine.

L⁵ is a single bond, a substituted or unsubstituted aromatic hydrocarbon ring group or fused aromatic hydrocarbon ring group having 6 to 30 carbon atoms, a substituted or unsubstituted cycloalkylene group having 5 to 30 carbon atoms or a substituted or unsubstituted aromatic heterocyclic ring group or fused aromatic heterocyclic ring group having 2 to 30 carbon atoms.
c is an integer of 0 to 2, d is an integer of 1 to 2 and e is an integer of 0 to 2, provided that c+e is 1 or more.

Specific Examples of the compound represented by the formula (20) are shown below.

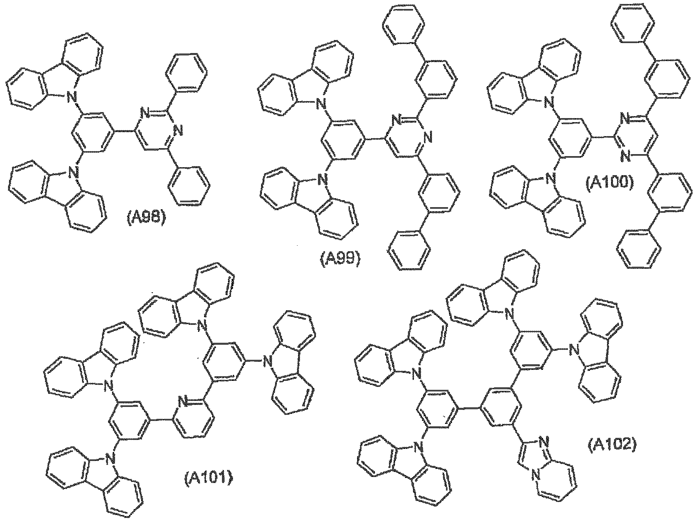

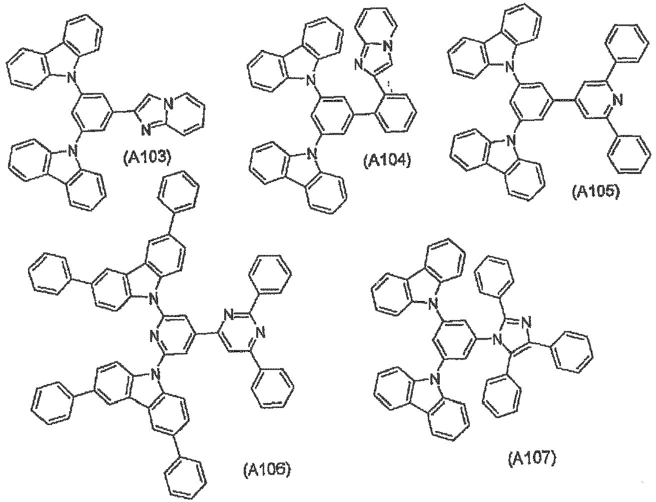

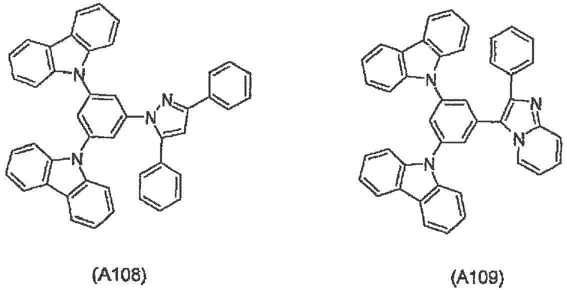

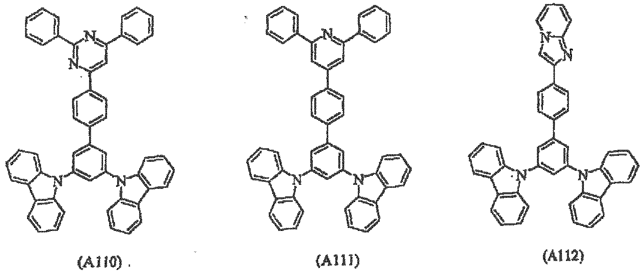

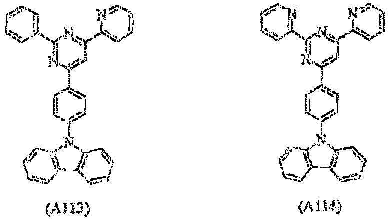

Specific Examples of the compound represented by the formula (21) are shown below.

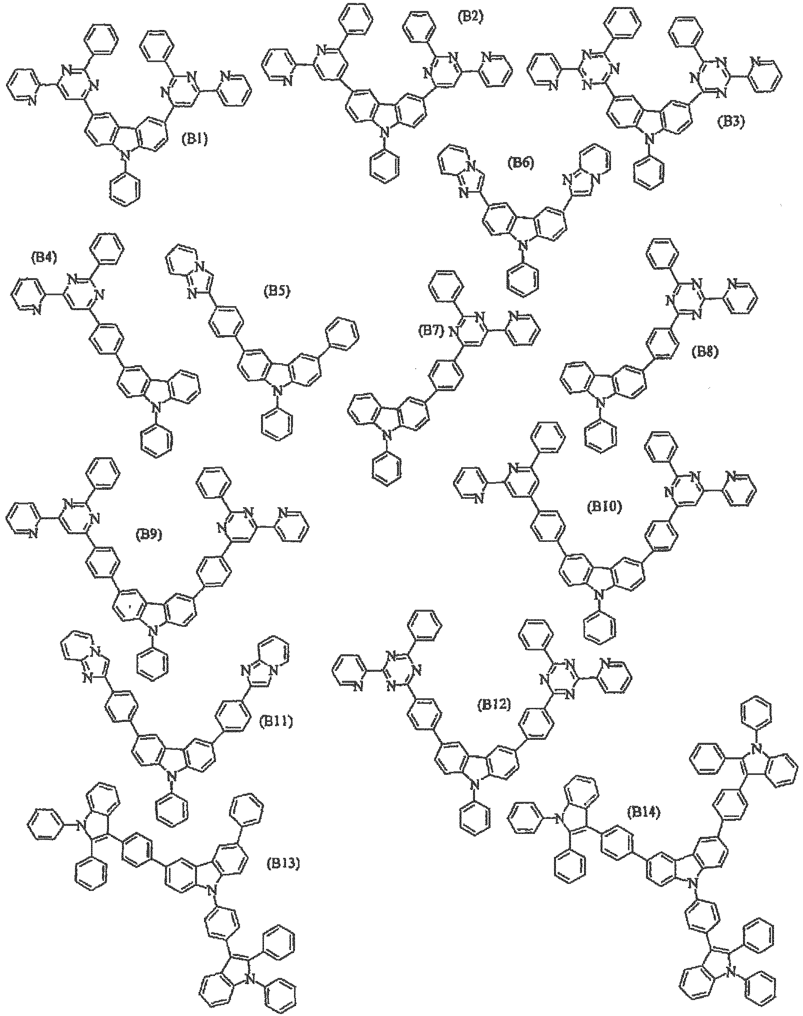

Furthermore, it is preferred that at least one layer of the organic thin film layers, preferably the emitting layer contain the compounds presented by the following formulas (30) to (33). These compounds are preferably used as a host material in the emitting layer.

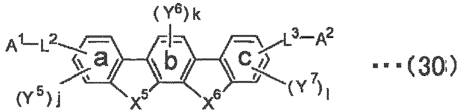

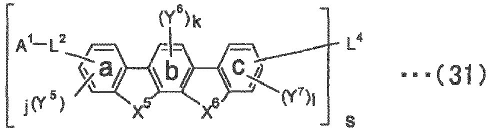

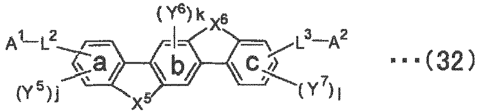

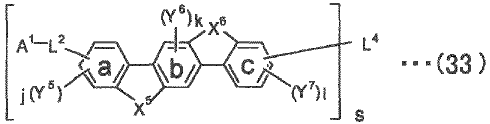

In the formulas (30) to (33), X⁵ and X⁶ are independently oxygen (O), sulfur (S), N-R¹ or CR²R³.
R¹, R² and R³ are independently an alkyl group having 1 to 20 carbon atoms, a substituted or unsubstituted cycloalkyl group having 3 to 20 ring carbon atoms, an aralkyl group having 7 to 24 carbon atoms, a silyl group or a substituted silyl group having 3 to 20 carbon atoms, a substituted or unsubstituted aromatic hydrocarbon ring group or fused aromatic hydrocarbon ring group having 6 to 24 ring carbon atoms or a substituted or unsubstituted aromatic heterocyclic group or fused aromatic heterocyclic group having 3o 24 ring carbon atoms, provided that when both X⁵ and X⁶ are N-R¹, at least one of R¹s is a substituted or unsubstituted monovalent fused aromatic heterocyclic group having 8 to24 ring atoms.

In the formulas (31) and (33), s is 2, 3 or 4, and the resulting compounds are a dimer, trimer or tetramer, which has L⁴ as a linking group, respectively.

In the formulas (30) to (33), L² is a single bond, an alkylene group having 1 to 20 carbon atoms, a substituted or unsubstituted cycloalkylene group having 3 to 20 ring carbon atoms, a substituted or unsubstituted silylene group having 2 to 20 carbon atoms, a substituted or unsubstituted divalent aromatic hydrocarbon ring group or fused aromatic hydrocarbon ring group having 6 to 24 ring carbon atoms, or a substituted or unsubstituted divalent aromatic heterocyclic group or fused aromatic heterocyclic group having 3 to 24 ring carbon atoms.

In the formulas (30) and (32), L³ is a single bond, an alkylene group having 1 to 20 carbon atoms, a substituted or unsubstituted cycloalkylene group having 3 to 20 ring carbon atoms, a substituted or unsubstituted silylene group having 2 to 20 carbon atoms, a substituted or unsubstituted divalent aromatic hydrocarbon ring group or fused aromatic hydrocarbon ring group having 6 to 24 ring carbon atoms or a substituted or unsubstituted divalent aromatic heterocyclic group or fused aromatic heterocyclic group having 3 to 24 ring carbon atoms.

In the formulas (31) and (33), when s is 2, L⁴ is a single bond, an alkylene group having 1 to 20 carbon atoms, a substituted or unsubstituted cycloalkylene group having 3 to 20 ring carbon atoms, a substituted or unsubstituted silylene group having 2 to 20 carbon atoms, a substituted or unsubstituted divalent aromatic hydrocarbon ring group or fused aromatic hydrocarbon ring group having 6 to 24 ring carbon atoms or a substituted or unsubstituted divalent aromatic heterocyclic group or fused aromatic heterocyclic group having 3 to 24 ring carbon atoms. When s is 3, L⁴ is the same as those given in the case where s is 2, except that the divalent groups are trivalent group corresponding thereto. When s is 4, L⁴ is the same as those given in the case where s is 2, except that the divalent groups are tetravalent group corresponding thereto.

In the formulas (30) to (33), A¹ is a hydrogen atom, a substituted or unsubstituted cycloalkyl group having 3 to 20 ring carbon atoms, a substituted or unsubstituted silyl group having 3 to 20 carbon atoms, a substituted or unsubstituted aromatic hydrocarbon ring group or fused aromatic hydrocarbon ring group having 6 to 24 ring carbon atoms, or a substituted or unsubstituted aromatic heterocyclic group or fused aromatic heterocyclic group having 3 to 24 ring carbon atoms.

In the formulas (30) and (32), A² is a hydrogen atom, a substituted or unsubstituted cycloalkyl group having 3 to 20 ring carbon atoms, a substituted or unsubstituted silyl group having 3 to 20 carbon atoms, a substituted or unsubstituted aromatic hydrocarbon ring group or fused aromatic hydrocarbon ring group having 6 to 24 ring carbon atoms or a substituted or unsubstituted aromatic heterocyclic group or fused aromatic heterocyclic group having 3 to 24 ring carbon atoms.

In the formulas (30) to (33), Y⁵, Y⁶ and Y⁷ are an alkyl group having 1 to 20 carbon atoms, a substituted or unsubstituted cycloalkyl group having 3 to 20 ring carbon atoms, an alkoxy group having 1 to 20 carbon atoms, an aralkyl group having 7 to 24 carbon atoms, a substituted or unsubstituted silyl group having 3 to 20 carbon atoms, a substituted or unsubstituted aromatic hydrocarbon ring group or fused aromatic hydrocarbon ring group having 6 to 24 ring carbon atoms, or a substituted or unsubstituted aromatic heterocyclic group or fused aromatic heterocyclic group having 3 to 24 ring carbon atoms. j and l are 0, 1, 2 or 3, and k is 0, 1 or 2.
In the formulas (30) to (33), A¹, A², L², L³ and L⁴ contain no carbonyl group.

Furthermore, it is preferred that at least one layer of the organic thin film layers, preferably the emitting layer contain the anthracene derivative represented by the following formula (40) or the pyrene derivative represented by the following formula (41). These derivatives are preferably used as a host material in the emitting layer.

### (Anthracene derivative)

The anthracene derivative represented by the formula (40) is the following compound.

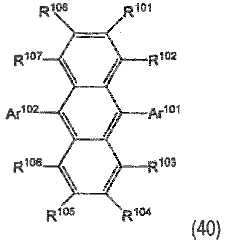

In the formula (40), Ar¹⁰¹ and Ar¹⁰² are independently a substituted or unsubstituted monocyclic group having 5 to 50 ring atoms, a substituted or unsubstituted fused ring group having 8 to 50 ring atoms or a group formed by combination of a monocyclic group and a fused ring group, and R¹⁰¹ to R¹⁰⁸ are independently an atom or a group selected from a hydrogen atom, a substituted or unsubstituted monocyclic group having 5 to 50 ring atoms, a substituted or unsubstituted fused ring group having 8 to 50 ring atoms, a group formed by combination of a monocyclic group and a fused ring group, a substituted or unsubstituted alkyl group having 1 to 50 carbon atoms, a substituted or unsubstituted cycloalkyl group having 3 to 50 ring carbon atoms, a substituted or unsubstituted alkoxy group having 1 to 50 carbon atoms, a substituted or unsubstituted aralkyl group having 7 to 50 carbon atoms, a substituted or unsubstituted aryloxy group having 6 to 50 ring carbon atoms, a substituted or unsubstituted silyl group, a halogen atom and a cyano group.

As specific examples of the monocyclic group having 5 to 50 (preferably 5 to 30, more preferably 5 to 20) ring atoms, aromatic groups such as a phenyl group, a biphenyl group, a terphenyl group, a quaterphenyl group and the like, and heterocyclic groups such as a pyridyl group, a pyradyl group, a pyrimidyl group, a triadinyl group, a furyl group, a thienyl group and the like, can be given preferably.
Among these, a phenyl group, biphenyl group or terphenyl group is preferable.

As specific examples of the fused ring group having 8 to 50 (preferably 8 to 30, more preferably 8 to 20) ring atoms, fused aromatic ring groups such as a naphthyl group, a phenanthryl group, an anthryl group, a chrysenyl group, a benzoanthryl group, a benzophenanthryl group, a triphenylenyl group, a benzochrysenyl group, an indenyl group, a fluorenyl group, a 9,9-dimethylfluorenyl group, a benzofluorenyl group, a dibenzofluorenyl group, a fluoranthenyl group, a benzofluoranthenyl group and the like, and fused heterocyclic groups such as a benzofuranyl group, a benzothiophenyl group, an indolyl group, a dibenzofuranyl group, a dibenzothiophenyl group, a carbazolyl group, a quinolyl group, a phenanthrolinyl group and the like, can be given preferably.
Among these, a naphthyl group, a phenanthryl group, an anthryl group, a 9,9-dimethylfluorenyl group, a fluoranthenyl group, a benzoanthryl group, a dibenzothiophenyl group, a dibenzofuranyl group or a carbazolyl group is preferable.

The specific examples of the alkyl group, substituted silyl group, cycloalkyl group and halogen atom in the formula (40) are the same as those of each in the above-mentioned formulas (1) to (3).

The alkoxy group is represented by -OY. Examples for Y include those described above for the alkyl group. The alkoxy group is a methoxy group or an ethoxy group, for example.

The aryloxy group is represented by -OZ. Examples for Z include those described above for the aryl group. The aryloxy group is a phenoxy group, for example.

The aralkyl group is represented by -Y-Z. Examples for Y include an alkylene group corresponding to those described above for the alkyl group. Examples for Z include those described above for the aryl group. The aralkyl group is preferably an aralkyl group having 7 to 50 carbon atoms, wherein the aryl part has 6 to 49 (preferably 6 to 30, more preferably 6 to 20, particular preferably 6 to 12) carbon atoms, and the alkyl part has 1 to 44 (preferably 1 to 30, more preferably 1 to 20, further preferably 1 to 10, particularly preferably 1 to 6) carbon atoms. For example, a benzyl group, phenylethyl group, or 2-phenylpropane-2-yl group can be given.

Preferable specific examples in the formula (40) are given below.
As preferable substituents of "substituted or unsubstituted" Ar¹⁰¹, Ar¹⁰², and R¹⁰¹ to R¹⁰⁸, a monocyclic group, fused ring group, alkyl group, cycloalkyl group, silyl group, alkoxy group, cyano group and halogen atom (in particular, fluorine) can be given. A monocyclic group and fused ring group are particularly preferable. The preferable specific examples are the same as those described above.

It is preferred that the anthracene derivative represented by the formula (40) be any of the following anthracene derivatives (A), (B) and (C), which is selected depending on the configuration or demanded properties of an organic EL device to which the derivative is applied.

### (Anthracene derivative (A))

This anthracene derivative is derivatives of formula (40) wherein Ar¹⁰¹ and Ar¹⁰² are independently a substituted or unsubstituted fused ring group having 8 to 50 ring atoms. This anthracene derivative can be classified into the case that Ar¹⁰¹ and Ar¹⁰² are the same substituted or unsubstituted fused ring group and the case that Ar¹⁰¹ and Ar¹⁰² are different substituted or unsubstituted fused ring groups.

Particularly preferred is the anthracene derivative of formula (40) wherein Ar¹⁰¹ and Ar¹⁰² are different (including difference in substituted positions) substituted or unsubstituted fused ring groups. Preferable specific examples of the fused ring are the same as those described above. Among those, a naphthyl group, a phenanthryl group, a benzanthryl group, a 9,9-dimethylfluorenyl group and a dibenzofuranyl group are preferable.

### (Anthracene derivative (B))

This anthracene derivative is derivatives of formula (40) wherein one of Ar¹⁰¹ and Ar¹⁰² is a substituted or unsubstituted monocyclic group having 5 to 50 ring atoms, and the other is a substituted or unsubstituted fused ring group having 8 to 50 ring atoms.
Preferable are anthracene derivatives wherein Ar¹⁰² is a naphthyl group, a phenanthryl group, a benzoanthryl group, a 9,9-dimethylfluorenyl group or a dibenzofuranyl group, and Ar¹⁰¹ is a phenyl group substituted by a monocyclic group or fused ring group.
Preferable specific examples of the monocyclic group and fused ring group are the same as those described above.
Preferable also are anthracene derivatives wherein Ar¹⁰² is a fused ring group, and A¹⁰¹ is an unsubstituted phenyl group. In this case, as the fused ring group, a phenanthryl group, a 9,9-dimethylfluorenyl group, a dibenzofuranyl group and a benzoanthryl group are particularly preferable.

### (Anthracene derivative (C))

This anthracene derivative is derivatives of formula (40) wherein Ar¹⁰¹ and Ar¹⁰² are independently a substituted or unsubstituted monocyclic group having 5 to 50 ring atoms.
Preferable are anthracene derivatives wherein both Ar¹⁰¹ and Ar¹⁰² are a substituted or unsubstituted phenyl group.
Further preferable are anthracene derivatives wherein Ar¹⁰¹ is an unsubstituted phenyl group, and Ar¹⁰² is a phenyl group having a monocyclic group and fused ring group as a substitutent, and anthracene derivatives wherein Ar¹⁰¹ and Ar¹⁰² are independently a phenyl group having a monocyclic group and fused ring group as a substitutent.
The preferable specific examples of the monocyclic group and fused ring group as a substituent are the same as those described above. As the monocyclic group as a substituent, a phenyl group and biphenyl group are more preferable. As the fused ring group as a substituent are a naphthyl group, phenanthryl group, 9,9-dimethylfluorenyl group, dibenzofuranyl group and benzoanthryl group are more preferable.

### (Pyrene derivative)

The pyrene derivative represented by the following formula (41) is the following compound.

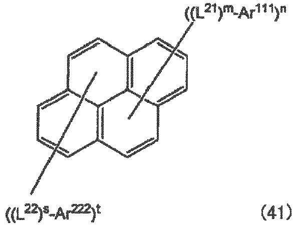

In the formula (41), Ar¹¹¹ and Ar²²² are independently a substituted or unsubstituted aryl group having 6 to 30 ring carbon atoms;
L²¹ and L²² are independently a substituted or unsubstituted divalent aryl group or heterocyclic group having 6 to 30 ring carbon atoms;
m is an integer of 0 to 1, n is an integer of 1 to 4, s is an integer of 0 to 1, and t is an integer of 0 to 3; and
L²¹ or Ar¹¹¹ is bonded at any of the 1- to 5-positions of the pyrene, and L²² or Ar²²² is bonded at any of the 6- to 10-positions of the pyrene.

L²¹ and L²² in the formula (41) are preferably a divalent aryl group composed of a substituted or unsubstituted phenylene group, a substituted or unsubstituted biphenylene group, a substituted or unsubstituted naphthylene group, a substituted or unsubstituted terphenylene group, a substituted or unsubstituted fluorenylene group, or a combination of these substituents.
Examples of the substituent are the same as those described above. The substituents of L²¹ and L²² are preferably an alkyl group having 1 to 20 carbon atoms.

m in the formula (41) is preferably an integer of 0 to 1. n in the formula (41) is preferably an integer of 1 to 2. s in the formula (41) is preferably an integer of 0 to 1.
t in the formula (41) is preferably an integer of 0 to 2.
The aryl groups of Ar¹¹¹ and Ar²²² are the same as those described above.
Preferable aryl groups are a substituted or unsubstituted aryl group having 6 to 20 ring carbon atoms, with a substituted or unsubstituted aryl group having 6 to 16 ring carbon atoms being more preferable. Preferable specific examples of the aryl groups include a phenyl group, a naphthyl group, a phenanthryl group, a fluorenyl group, a biphenyl group, an anthryl group and a pyrenyl group.
When the derivatives represented by the above formulas (20) to (22), (30) to (33), (40) and (41) are used as a host material in an emitting layer, it is possible to use two or more of these derivatives in combination.

The emitting layer may contain a luminous dopant (phosphorescent dopant and/or fluorescent dopant) in addition to an emitting material.

The fluorescent dopant is a compound which can emit light from singlet excitons. It is preferred that the fluorescent dopant be selected from amine compounds, aromatic compounds, chelate complexes such as tris(8-quinolinolato)aluminum complex, coumalin derivatives, tetraphenylbutadiene derivatives, bisstyrylarylene derivatives, oxadiazole derivatives and the like depending on demanded emitting color. More preferable are styrylamine compounds, styryldiamine compounds, arylamine compounds and aryldiamine compounds. Still more preferable is fused polycyclic amine derivatives. These fluorescent dopants can be used singly or in combination thereof.

As a preferable fused polycyclic amine derivative, one represented by the following formula (50) can be given.

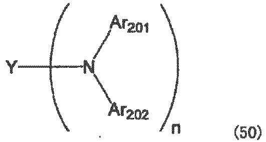

In the formula (50), Y is a substituted or unsubstituted fused aryl group having 10 to 50 ring carbon atoms; and
Ar₂₀₁ and Ar₂₀₂ are independently a substituted or unsubstituted aryl group having 6 to 50 ring carbon atoms or a substituted or unsubstituted heterocyclic group having 5 to 50 ring atoms.

The fused aryl group is an aryl group formed by condensation of 2 or more ring structures among the above-mentioned aryl groups.
The fused aryl group is a fused aryl group having 10 to 50 (preferably 10 to 30, more preferably 10 to 20) ring carbon atoms. Among the specific examples of aryl group mentioned above, preferable are a naphthyl group, an anthryl group, a pyrenyl group, a phenanthryl group, a fluorenyl group, a fluorantenyl group and a naphtacenyl group, etc.

Specific Examples of Y include the fused aryl groups described above. Preferable are a substituted or unsubstituted anthryl group, a substituted or unsubstituted pyrenyl group, and a substituted or unsubstituted chrysenyl group.

Preferable examples of Ar₂₀₁ and Ar₂₀₂ include a substituted or unsubstituted phenyl group and a substituted or unsubstituted dibenzofuranyl group. Preferable examples of the substituent of Ar₂₀₁ and Ar₂₀₂ include an alkyl group, a cyano group, and a substituted or unsubstituted silyl group.
n is an integer of 1 to 4. n is preferably an integer of 1 to 2.

As the styrylamine compounds and styryldiamine compounds, the compounds represented by the following formulas (51) and (52) are preferable.

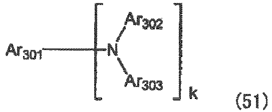

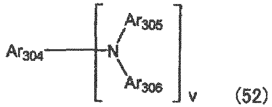

In the formula (51), Ar₃₀₁ is a k-valent group which corresponds to phenyl, naphthyl, biphenyl, terphenyl, stilbene, styrylaryl, or distyrylaryl. Ar₃₀₂ and Ar₃₀₃ are independently an aryl group having 6 to 20 ring carbon atoms. Ar₃₀₁, Ar₃₀₂ and Ar₃₀₃ may be substituted.
k is an integer of 1 to 4. Among these, k is preferably an integer of 1 to 2. Any one of Ar₃₀₁ to Ar₃₀₃ is a group containing a styryl group. At least one of Ar₃₀₂ and Ar₃₀₃ is more preferably substituted with a styryl group.
Here, as the aryl group having 6 to 20 ring carbon atoms, the above-mentioned aryl groups can be given specifically. Among these, a phenyl group, a naphthyl group, an anthranil group, a phenanthryl group, a terphenyl group and the like are preferable.

Ar₃₀₄ to Ar₃₀₆ in the formula (52) is a substituted or unsubstituted v-valent aryl group having 6 to 40 ring carbon atoms. v is an integer of 1 to 4. Among these, v is preferably an integer of 1 to 2.
Here, as the aryl group having 6 to 40 ring carbon atoms in the formula (52), the above-mentioned aryl groups can be given specifically. Preferable is a naphthyl group, anthranil group, crysenyl group or pyrenyl group.

Preferable substituents on the aryl group include an alkyl group having 1 to 6 carbon atoms, an alkoxy group having 1 to 6 carbon atoms, an aryl group having 6 to 40 ring carbon atoms, an amino group substituted with an aryl group having 6 to 40 ring carbon atoms, an ester group having an aryl group having 5 to 40 ring carbon atoms, an ester group having an alkyl group having 1 to 6 carbon atoms, a cyano group, a nitro group, a halogen atom and the like.

The phosphorescent dopant is a compound which can emit light from triplet excitons.
The phosphorescent dopant includes a metal complex. It is preferred that the metal complex have a metal atom selected from Ir, Pt, Os, Au, Cu, Re and Ru, and a ligand. In particular, the ligand has preferably an ortho metal bonding.
It is preferred that the phosphorescent dopant be a compound containing a metal atom selected from Ir, Os and Pt in view of high phosphorescent quantum yield and more improved external quantum efficiency of the light emitting device. More preferable are a metal complex such as an iridium complex, an osmium complex, a platinum complex and the like. In particular, an iridium complex and a platinum complex are more preferable, and an ortho metalated iridium complex is most preferable.
Specific examples of preferable metal complex are shown below.

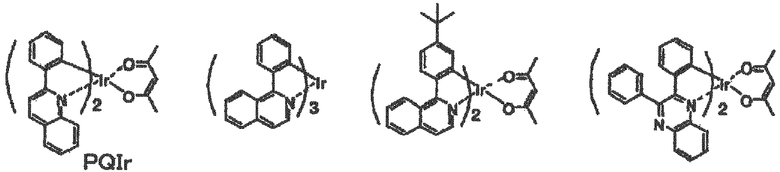

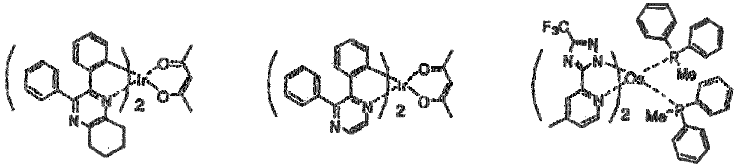

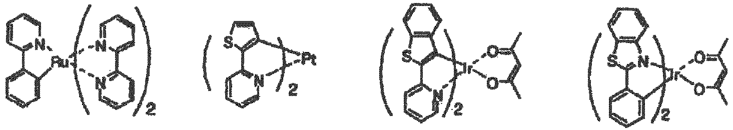

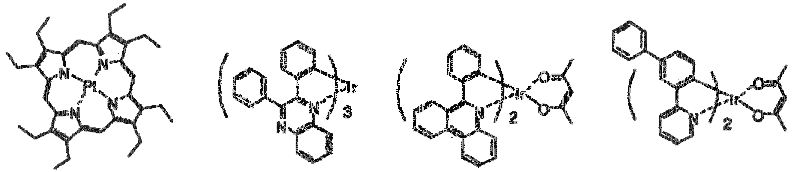

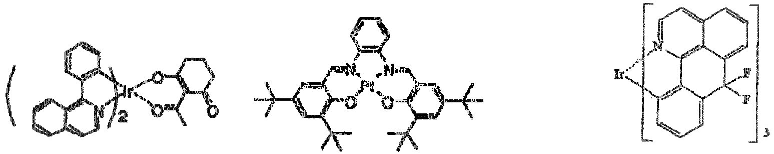

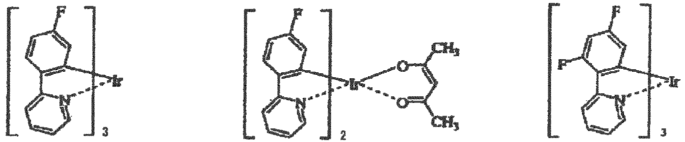

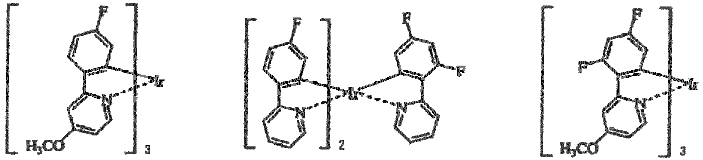

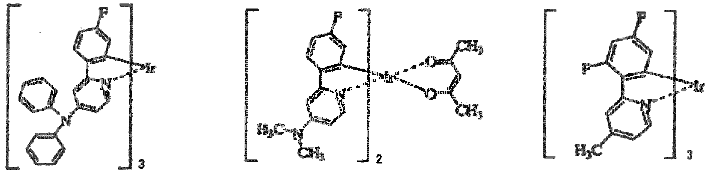

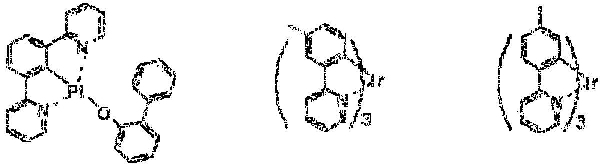

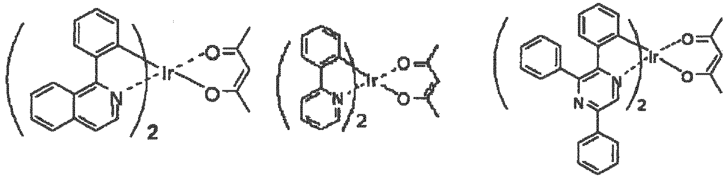

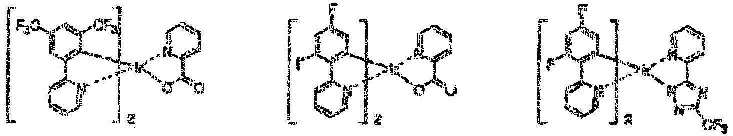

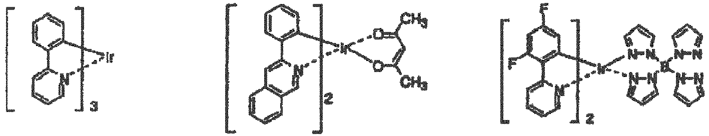

**Ir(ppy)₃**

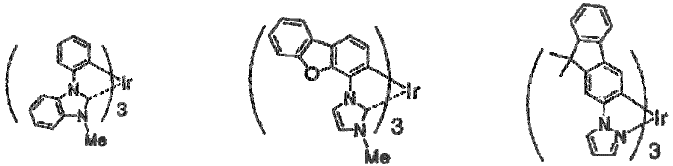

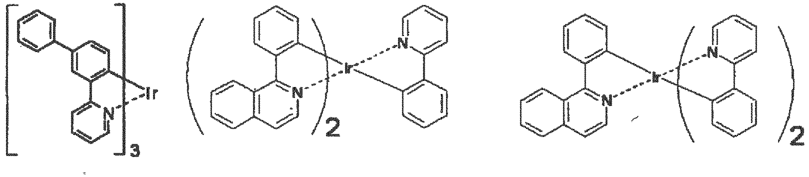

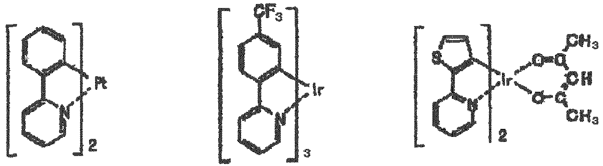

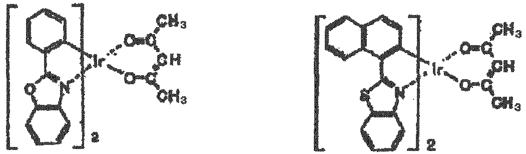

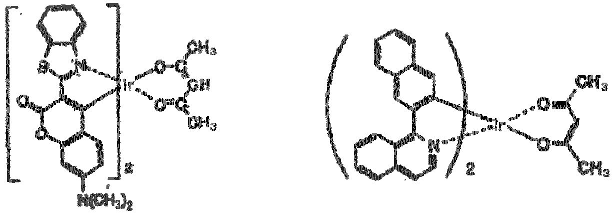

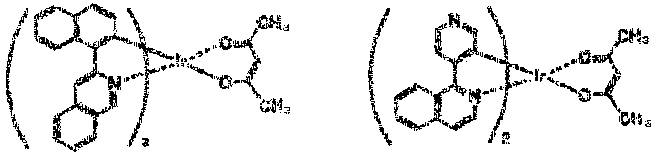

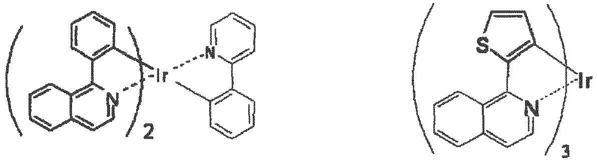

A preferable electron-injecting and/or -transporting material is a compound which can transport electrons, has an electron injection effect from a cathode and an excellent electron injection effect into an emitting layer or an emitting material, and exhibits an excellent thin-film forming ability.

In the organic EL device of the invention, more effective electron-injecting material is a metal complex compound and a nitrogen-containing heterocyclic derivative.
Examples of the metal complex compound include, but not limited to, 8-hydroxyquinolinatelithium, bis(8-hydroxyquinolinato)zinc, tris(8-hydroxyquinolinato)aluminum, tris(8-hydroxyquinolinato)gallium, bis(10-hydroxybenzo[h]quinolinato)berylium, bis(10-hydroxybenzo[h]quinolinato)zinc and the like.

Preferable examples of the nitrogen-containing heterocyclic derivative include oxazole, thiazole, oxadiazole, thiadiazole, triazole, pyridine, pyrimidine, triazine, phenanthroline, benzimidazole, imdazopyridine and the like. Among these, benzimidazole derivatives, phenanthroline derivatives and imidazopyridine derivatives are preferable.
In a preferable organic EL device of the invention, a dopant is also contained in these electron-injecting materials. It is more preferred that the neighborhood of the interface between an organic layer and cathode be doped with a dopant represented by an alkali metal in order to facilitate accepting of electrons from the cathode.
As the dopant, a donor metal, a donor metal compound and a donor metal complex can be given. These reducing dopants can be used singly or in combination of two or more.

It is preferred that the organic EL device of the invention have at least an electron-transporting layer as an organic thin film layer, and the electron-transporting layer contain a nitrogen-containing heterocyclic derivative represented by any of the following formulas (60) to (62).

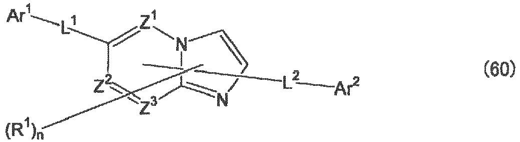

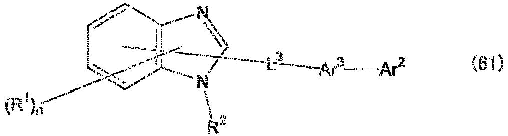

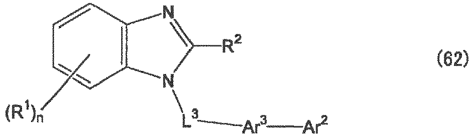

In the formulas (60) to (62), Z¹, Z² and Z³ are independently a nitrogen atom, a carbon atom or C-H.
R¹ and R² are independently a substituted or unsubstituted aryl group having 6 to 50 ring carbon atoms, a substituted or unsubstituted heteroaryl group having 3 to 50 ring atoms, an alkyl group having 1 to 20 carbon atoms, or an alkyl group having 1 to 20 carbon atoms which is substituted with a halogen atom or an alkoxy group having 1 to 20 carbon atoms. Preferable is a substituted or unsubstituted aryl group having 6 to 50 ring carbon atoms such as a phenyl group.
n is an integer of 0 to 5, when n is an integer of 2 or more, R¹s may be the same or different, or two adjacent R¹s may be bonded to each other to form a substituted or unsubstituted aromatic hydrocarbon ring.
Ar¹ is a substituted or unsubstituted aryl group having 6 to 50 ring carbon atoms or a substituted or unsubstituted heteroaryl group having 3 to 50 ring atoms.
Ar² is a hydrogen atom, an alkyl group having 1 to 20 carbon atoms, an alkyl group having 1 to 20 carbon atoms which is substituted with a halogen atom, an alkoxy group having 1 to 20 carbon atoms, a substituted or unsubstituted aryl group having 6 to 50 ring carbon atoms or a substituted or unsubstituted heteroaryl group having 3 to 50 ring atoms.
Meanwhile, one of Ar¹ and Ar² is a substituted or unsubstituted fused aromatic hydrocarbon ring group having 10 to 50 ring carbon atoms (a naphthyl group, for example) or a substituted or unsubstituted fused aromatic heterocyclic group having 9 to 50 ring atoms.
Ar³ is an arylene group having 6 to 50 ring carbon atoms (an anthracenylene group, for example) or a substituted or unsubstituted heteroarylene group having 3 to 50 ring atoms.
L¹, L² and L³ are independently a single bond, a substituted or unsubstituted arylene group having 6 to 50 ring carbon atoms (a phenylene group, a fluorenylene group, for example) or a substituted or unsubstituted hetero fused ring group having 9 to 50 ring atoms.

For the members such as a substrate, an anode, a cathode and the like of the organic EL device, in addition to those described above, it is possible to select appropriately and use known materials described in documents such as WO2009/107596A1, WO2009/081857A1, US2009/0243473A1, US2008/0014464A1, US2009/0021160A1 and the like.

### EXAMPLES

The invention will be explained in more detail with reference to Examples below, but is not limited to these.
The structures of Intermediate produced in Synthesis Examples 1 to 14 below are as follows:

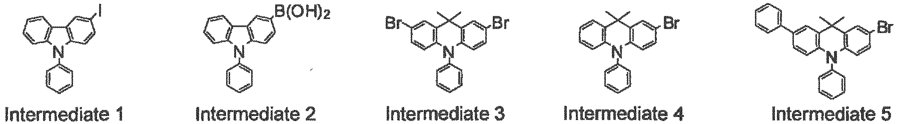

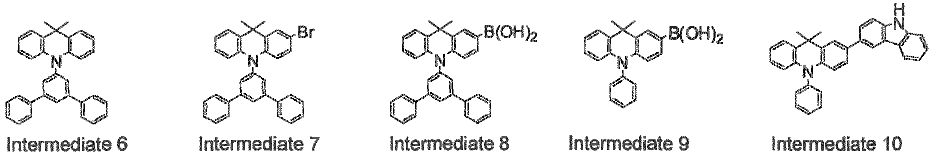

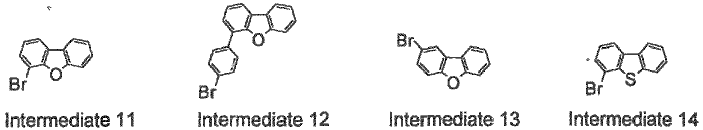

### Synthesis Example 1 (Synthesis of Intermediate 1)

17.7g of 9-phenylcarbazole, 6.03g of potassium iodide, 7.78g of potassium iodate, 5.90 mL of sulfuric acid and ethanol were placed and reacted at 75 °C for 2 hours.
After cooling, water and ethyl acetate was added thereto. After the resulting mixture was separated and extracted, an organic phase thereof was washed with sodium bicarbonate water and water, and concentrated. The crude product obtained was purified by silica-gel chromatography (toluene), and the solids obtained were dried under reduced pressure to obtain 21.8g of white solids. The white solids were identified as Intermediate 1 by FD-MS analysis.

### Synthesis Example 2 (Synthesis of Intermediate 2)

Under a flow of argon, dehydrated toluene and dehydrated ether were added to Intermediate 1 (13.1g), and the resulting mixture was cooled to -45 °C. 25 mL of a hexane solution of n-butyllithium (1.58M) was dropped thereto, and the mixture was heated to -5 °C over an hour while stirring. After cooled to -45 °C again, 25 mL of boronic acid triisopropyl ester was dropped slowly, followed by reaction for 2 hours.
After the resultant was returned to room temperature, a 10% diluted hydrochloric acid solution was added and the resulting mixture was stirred to extract an organic phase. After washed with saturated saline, the organic layer was dried with anhydrous magnesium sulfate. It was separated by filtration, followed by concentration. The solids obtained were purified by silica-gel chromatography (toluene) and the solids obtained were washed with n-hexane and dried under reduced pressure to obtain 7.10g of solids. The solids were identified as Intermediate 2 by FD-MS analysis.

### Synthesis Example 3 (Synthesis of Intermediate 3)

Under argon atmosphere, dimethylformamide (DMF) (350 mL) was added to 9,9-dimethyl-10-phenyl-9,10-dihydroacridine (28.5g, 100 mmol) and N-bromosuccinimide (NBS) (35.6g, 200 mmol), and the resulting mixture was stirred at room temperature for 8 hours. After the reaction was completed, the sample was transferred to a separating funnel, and water (500 mL) was added thereto, followed by extraction with ethyl acetate. The sample was purified by column chromatography to obtain 35.4g of white solids. The solids were identified as Intermediate 3 by FD-MS analysis.

### Synthesis Example 4 (Synthesis of Intermediate 4)

Under argon atmosphere, DMF (350 mL) was added to 9,9-dimethyl-10-phenyl-9,10-dihydroacridine (28.5g, 100 mmol) and NBS (17.8g, 100 mmol), and the resulting mixture was stirred at room temperature for 8 hours. After the reaction was completed, the sample was transferred to a separating funnel, and water (500 mL) was added thereto, followed by extraction with ethyl acetate. The sample was purified by column chromatography to obtain 18.4g of white solids. The solids were identified as Intermediate 4 by FD-MS analysis.

### Synthesis Example 5 (Synthesis of Intermediate 5)

Under argon atmosphere, 10 mL of toluene, 10 mL of 1,2-dimethoxyethane and 5 mL of a 2M aqueous solution of sodium carbonate were added to Intermediate 3 (4.4g), 1.2g of phenylboronic acid and 92 mg of tetrakis(triphenylphosphine)palladium(0). The resulting mixture was heated under reflux for 10 hours.
A filtration was conducted immediately after the reaction was completed, followed by removal of water layer. After dried with sodium sulfate, the organic layer was concentrated. The residue was purified by silica-gel column chromatography to obtain 2.1g of white crystals. The crystals were identified as Intermediate 5 by FD-MS analysis.

### Synthesis Example 6 (Synthesis of Intermediate 6)

Under a flow of argon, 2.1g of 9,9-dimethyl-9,10-dihydroacridine, 3.1g of 1-bromo-3,5-diphenylbenzene, 1.3g of t-butoxysodium, 46 mg of tris(dibenzilideneacetone)dipalladium, 29 mg of tri-tert-butylphosphonium tetrafluoroborate and 50 mL of dehydrated toluene were placed and reacted at 80 °C for 8 hours.
After cooling, 500 mL of water was added thereto, the mixture was filtered through celite. The filtrate was extracted with toluene and dried with anhydrous magnesium sulfate. The resultant was concentrated under reduced pressure. The crude product obtained was purified by column chromatography, and was recrystallized from tolunene. After removal by filtration, drying was conducted to obtain 4.0g of white powder. The powder was identified as Intermediate 6 by FD-MS analysis.

### Synthesis Example 7 (Synthesis of Intermediate 7)

6.2g of white powder was obtained in the same manner as in Synthesis Example 4, except that 10.0g of Intermediate 6 was used instead of 9,9-dimethyl-10-phenyl-9,10-dihydroacridine. The powder was identified as Intermediate 7 by FD-MS analysis.

### Synthesis Example 8 (Synthesis of Intermediate 8)

3.2g of white solids was obtained in the same manner as in Synthesis Example 2, except that 6.2g of Intermediate 7 was used instead of Intermediate 1. The solids were identified as Intermediate 8 by FD-MS analysis.

### Synthesis Example 9 (Synthesis of Intermediate 9)

Under argon atmosphere, 200 ml of dehydrated xylene was added to Intermediate 4 (32.7g, 89.8 mmol) and the mixture was cooled to -30 °C. 60 ml (96.0 mmol) of a 1.6M n-butyllithium-hexane solution was added, and the mixture was reacted for 1 hour. After the resultant was cooled to -70 °C, 45.9g (244.0 mmol) of triisopropyl borate was added. The reaction solution was heated gradually, and stirred at room temperature for an hour. Then, 64 ml of a 10% hydrochloric solution was added, and the mixture was stirred. After the reaction was completed, extraction with ethyl acetate was conducted. The organic layer was cleaned with water, dried with Na₂SO₄, and concentrated. The residue was washed with hexane to obtain 17.7g of white solids.

### Synthesis Example 10 (Synthesis of Intermediate 10)

Under argon atmosphere, 40 ml (80.0 mmol) of a 2M aqueous solution of Na₂CO₃, 200 ml of dioxane and 0.33g (0.4 mmol) of PdCl₂ (dppf) were added to 9.8g (40.0 mmol) of 3-bromocarbazole and Intermediate 9 (14.5g, 44.0 mmol), and the resulting mixture was heated under reflux while stirring for 12 hours.
After the reaction was completed, the resultant was cooled to room temperature. The sample was transferred to a separating funnel, and water (100 ml) was added thereto, followed by extraction with dichloromethane. The extracted sample was dried with MgSO₄, followed by filtration and concentration. The residue was purified by silica-gel column chromatography to obtain 9.0g of white solids. The solids were identified as Intermediate 10 by FD-MS analysis.

### Synthesis Example 11 (Synthesis of Intermediate 11)

Under argon atmosphere, 600 ml of dehydrated tetrahydrofuran was added to 78.0g (0.46 mol) of dibenzofuran, and the mixture was cooled to -30 °C. Thereto, 300 ml (0.50 mol) of a 1.65M n-butyllithium-hexane solution was dropped, and the resulting mixture was heated to room temperature while stirring over 1 hour. After stirred at room temperature for 5 hours, the mixture was cooled to -60 °C, and 60 ml (0.70 mol) of 1,2-dibromoethane was dropped thereto over 1 hour.
After stirred at room temperature for 15 hours, the resulting mixture was poured to 1000 ml of iced water, followed by extraction with dichloromethane. After washed with saturated saline, the organic layer was dried with MgSO₄, followed by filtration and concentration. The concentrated residue was purified by silica-gel chromatography, and washed with tetrahydrofuran/methanol to obtain 70g of solids. The solids were identified as Intermediate 11 by FD-MS analysis.

### Synthesis Example 12 (Synthesis of Intermediate 12)

Under argon atmosphere, 150 ml of toluene, 150 ml of dimethoxyethane and 150 ml (300.0 mmol) of a 2M aqueous solution of Na₂CO₃ were added to 28.3g (100.0 mmol) of 4-iodobromobenzene and 22.3g (105.0 mmol) of dibenzofuran-4-boronic acid and 2.31g (2.00 mmol) of Pd[PPh₃]₄. The resulting mixture was heated under reflux while stirring for 10 hours.
After the reaction was completed, the sample was transferred to a separating funnel, followed by extraction with dichloromethane. The organic layer was dried with MgSO₄, followed by filtration and concentration. The concentrated residue was purified by silica-gel column chromatography to obtain 26.2g of white solids. The solids were identified as Intermediate 12 by FD-MS analysis.

### Synthesis Example 13 (Synthesis of Intermediate 13)

Under nitrogen atmosphere, 1000 ml of acetic acid was added to 150g (0.89 mol) of dibenzofuran, followed by heating and dissolution. Furthermore, 188g (1.18 mol) of bromine was added thereto dropwise, and then the resulting mixture was stirred at room temperature for 20 hours. The precipitated solids were filtered out, and washed with acetic acid and water sequentially. The recrystallization of the crude product from methanol was repeated several times to obtain 66.8g of white solids. The solids were identified as Intermediate 13 by FD-MS analysis.

### Synthesis Example 14 (Synthesis of Intermediate 14)

Under argon atmosphere, 480 ml of dehydrated tetrahydrofuran was added to 48.2g (261.6 mmol) of dibenzothiophene, and the mixture was cooled to -30 °C. Thereto, 164 ml (262.0 mol) of a 1.60M n-butyllithiumhexane solution was dropped, and the resulting mixture was heated to room temperature while stirring over 1 hour. After stirred at room temperature for 3 hours, the mixture was cooled to -60 °C, and a solution obtained by dissolving 73.7g (393 mmol) of 1,2-dibromoethane in 50 ml of dehydrated tetrahydrofuran was dropped thereto for 1 hour.
After stirred at room temperature for 15 hours, the resulting mixture was poured to 400 ml of iced water, followed by extraction with toluene. After washed with saturated saline, the organic layer was dried with MgSO₄, followed by filtration and concentration. The concentrated residue was purified by silica-gel chromatography. The recrystallization of the crude product from heptane was repeated several times to obtain 33.1g of white solids. The solids were identified as Intermediate 14 by FD-MS analysis.

The structures of the aromatic heterocyclic derivatives according to the invention prepared in Examples 1 to 8 are as follows:

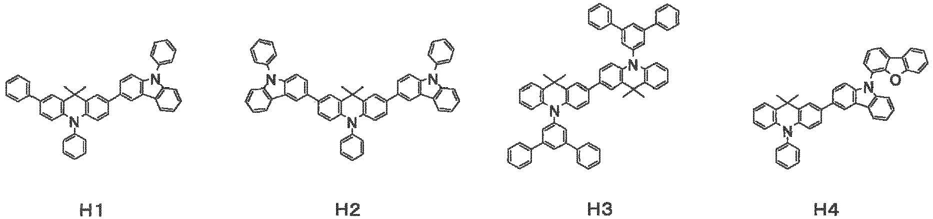

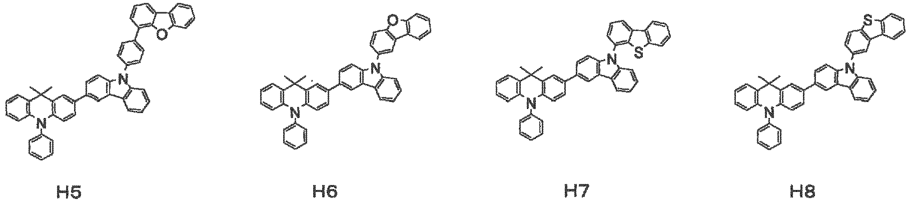

### Example 1 (Preparation of Aromatic heterocyclic derivative (H1))

10 mL of toluene, 10 mL of 1,2-dimethoxyethane and 5 mL of a 2M aqueous solution of sodium carbonate were added to 4.4g of Intermediate 5, 3.0g of Intermediate 2 and 92 mg of tetrakis(triphenylphosphine)paradium(0) under an argon atmosphere. The mixture was heated under reflux for 10 hours.
Immediately after the reaction was completed, the mixture was filtered, and then the aqueous phase was removed. The organic phase was dried with sodium sulfate and then concentrated. The residue was purified by silica-gel column chromatography to obtain 4.0g of white crystals. The white powder was identified as Aromatic heterocyclic derivative (H1) by FD-MS analysis.

### Example 2 (Preparation of Aromatic heterocyclic derivative (H2))

Reaction was conducted in a similar way to Example 1 except that 4.4g of Intermediate 3 and 6.3g of Intermediate 2 were used instead of Intermediate 5 to obtain 5.4g of white powder. The white powder was identified as Aromatic heterocyclic derivative (H2) by FD-MS analysis.

### Example 3 (Preparation of Aromatic heterocyclic derivative (H3))

Reaction was conducted in a similar way to Example 1 except that 5.2g of Intermediate 7 was used instead of Intermediate 5 and 4.8g of Intermediate 8 was used instead of Intermediate 2 to obtain 6.3g of white powder. The white powder was identified as Aromatic heterocyclic derivative (H3) by FD-MS analysis.

### Example 4 (Preparation of Aromatic heterocyclic derivative (H4))

Under an argon atmosphere, 50 ml of anhydrous xylene was added to Intermediate 10 (4.5g, 10.0 mmol), Intermediate 11 (2.5g, 10.0 mmol), Pd₂(dba)₃ (0.14g, 0.15 mmol), P(tBu)₃HBF₄ (0.087g, 0.3 mmol) and sodium t-butoxide (1.9g, 20.0 mmol). The mixture was heated under reflux for 8 hours.
After the reaction was completed, the reaction solution was cooled to 50 °C. The solution was filtered through celite and silica gel, and the filtrate was concentrated. The concentrated residue obtained was purified by silica-gel column chromatography to obtain white solids. The crude product was recrystallized from toluene to obtain 3.1g of white crystals. The white crystals were identified as Aromatic heterocyclic derivative (H4) by FD-MS analysis.

### Example 5 (Preparation of Aromatic heterocyclic derivative (H5))

Reaction was conducted in a similar way to Example 4 except that 3.2g of Intermediate 12 was used instead of Intermediate 11 to obtain 4.8g of white powder. The white powder was identified as Aromatic heterocyclic derivative (H5) by FD-MS analysis.

### Example 6 (Preparation of Aromatic heterocyclic derivative (H6))

Reaction was conducted in a similar way to Example 4 except that 2.5g of Intermediate 1.3 was used instead of Intermediate 11 to obtain 3.7g of white powder. The white powder was identified as Aromatic heterocyclic derivative (H6) by FD-MS analysis.

### Example 7 (Preparation of Aromatic heterocyclic derivative (H7))

Reaction was conducted in a similar way to Example 4 except that 2.6g of Intermediate 14 was used instead of Intermediate 11 to obtain 1.9g of white powder. The white powder was identified as Aromatic heterocyclic derivative (H7) by FD-MS analysis.

### Example 8 (Preparation of Aromatic heterocyclic derivative (H8))

Reaction was conducted in a similar way to Example 4 except that 2.6g of 2-bromo dibenzothiophene was used instead of Intermediate 11 to obtain 3.2g of white powder. The white powder was identified as Aromatic heterocyclic derivative (H8) by FD-MS analysis.

### Example 1-1 (Production of Organic EL device)

A glass substrate of 25 mm by 75 mm by 1.1 mm with an ITO transparent electrode (GEOMATEC CO., LTD.) was subjected to ultrasonic cleaning in isopropyl alcohol for 5 minutes, and cleaning with ultraviolet rays and ozone for 30 minutes.
The cleaned substrate with transparent electrode lines was mounted on a substrate holder in a vacuum deposition device. First, the following electron-acceptor compound (A) was deposited to form a 5 nm-thick A film so as to cover the surface of the transparent electrode on which the transparent electrode lines were formed. The following aromatic amine derivative (X1) was deposited as a first hole-transporting material on the A film to form a 120 nm-thick first hole-transporting layer. Following formation of the first hole-transporting layer, the aromatic heterocyclic derivative (H1) obtained in Example 1 was deposited as a second hole-transporting material to form a 20 nm-thick second hole-transporting layer.
On the second hole-transporting layer, Compound (B) as a phosphorescent host and Ir(ppy)₃ as a phosphorescent dopant were co-deposited in a thickness of 40 nm to obtain a phosphorescent emitting layer. The concentration of Ir(ppy)₃ was 15 mass %.
Subsequently, on the phosphorescent emitting layer, Compound (C) with a thickness of 20 nm, LiF with a thickness of 1 nm and metal Al with a thickness of 80 nm were stacked sequentially to obtain a cathode. LiF as an electron-injecting electrode was formed at a film forming rate of 1 Å/min.

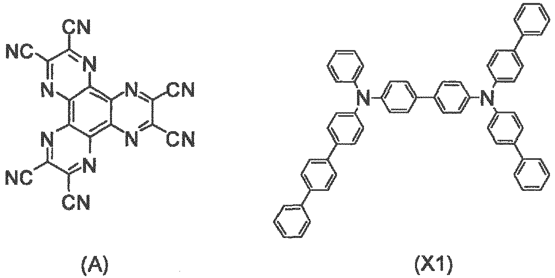

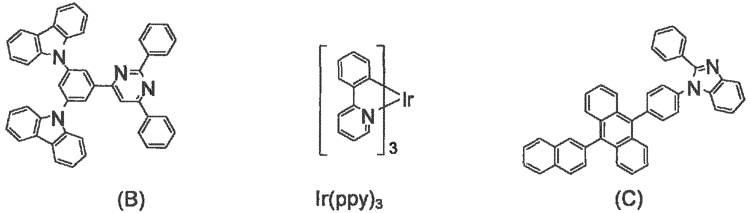

### (Luminescent performance evaluation of organic EL device)

By allowing the organic EL devices produced as above to be emitted by DC driving, the luminance (L) and current density were measured to determine the luminous efficiency (cd/A) and driving voltage (V) at a current density of 1 mA/cm².
Furthermore, the device lifetime (half life) at a 10000 cd/m² of initial luminance was determined. Table 1 shows the results.

### Examples 1-2 to 1-8 (Production and Luminescent performance evaluation of Organic EL device)

Organic EL devices were produced and evaluated in the same manner as in Example 1-1, except that the following aromatic heterocyclic derivatives shown in Table 1 were used instead of Aromatic heterocyclic derivative (H1) as the second hole-transporting material. Table 1 shows the results.

### Comparative Examples 1-1 and 1-2 (Production and Luminescent performance evaluation of Organic EL device)

Organic EL devices were produced and evaluated in the same manner as in Example 1-1, except that the following Comparative compounds 1 and 2 were used instead of Aromatic heterocyclic derivative (H1) as the second hole-transporting material in Example 1. Table 1 shows the results.

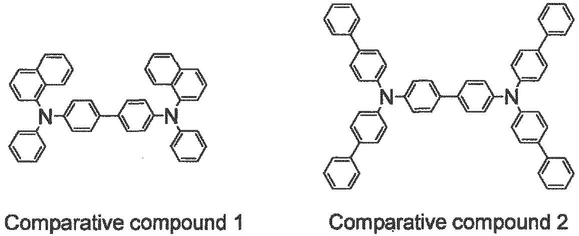

**Table 1**

| | | Second Hole-transporting material | Measurement results | | |
|---|---|---|---|---|---|
| | | | Luminous efficiency (cd/A) | Driving voltage (V) | Half life (hour) |
| | | | @1mA/cm² | @1mA/cm² | |
| Exam. | 1-1 | H1 | 72 | 4.5 | 300 |
| | 1-2 | H2 | 70 | 4.8 | 350 |
| | 1-3 | H3 | 72 | 4.6 | 330 |
| | 1-4 | H4 | 74 | 4.7 | 330 |
| | 1-5 | H5 | 73 | 4.6 | 330 |
| | 1-6 | H6 | 72 | 4.3 | 300 |
| | 1-7 | H7 | 75 | 4.7 | 310 |
| | 1-8 | H8 | 75 | 4.3 | 330 |
| Com. Ex. | 1-1 | Comparative compound 1 | 55 | 4.4 | 200 |
| | 1-2 | Comparative compound 2 | 58 | 4.5 | 200 |

As shown in Table 1, the organic EL devices using the aromatic heterocyclic derivative according to the invention in the hole-transporting layer could have a higher luminous efficiency and a longer device life compared with the organic EL devices using the comparative compounds.

### Example 2-1 (Production of Organic EL device)

A glass substrate of 25 mm by 75 mm by 1.1 mm with an ITO transparent electrode (GEOMATEC CO., LTD.) was subjected to ultrasonic cleaning in isopropyl alcohol for 5 minutes, and cleaning with ultraviolet rays and ozone for 30 minutes.
The cleaned substrate with transparent electrode lines was mounted on a substrate holder in a vacuum deposition device. First, the above-mentioned electron-acceptor compound (A) was deposited to form a 5 nm-thick A film so as to cover the surface of the transparent electrode on which the transparent electrode lines were formed. The above-mentioned aromatic amine derivative (X1) was deposited as a first hole-transporting material on the A film to form a 70 nm-thick first hole-transporting layer. Subsequent to the forming of the first hole-transporting layer, the aromatic heterocyclic derivative (H1) obtained in Example 1 was deposited as a second hole-transporting material to form a 20 nm-thick second hole-transporting layer.
Furthermore, on the second hole-transporting layer, Compound (B) as a phosphorescent host and Flrpic as a phosphorescent dopant were co-deposited in a thickness of 40 nm to obtain a phosphorescent emitting layer. The concentration of Flrpic was 15 mass %.
Subsequently, on the phosphorescent emitting layer, Compound (C) with a thickness of 20 nm, LiF with a thickness of 1 nm and metal Al with a thickness of 80 nm were stacked sequentially to obtain a cathode. LiF as an electron-injecting electrode was formed at a film forming rate of 1 Å/min

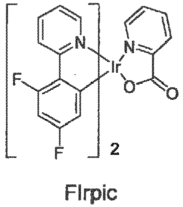

### (Luminescent performance evaluation of organic EL device)

By allowing the organic EL devices produced as above to be emitted by DC driving, the luminance (L) and current density were measured to determine the luminous efficiency (cd/A) and driving voltage (V) at a current density of 1 mA/cm².
Furthermore, the device lifetime (half life) at a 10000 cd/m² of initial luminance was determined. Table 2 shows the results.

### Examples 2-2 to 2-3

Organic EL devices were produced and evaluated in the same manner as in Example 2-1, except that Compound (H2) and (H3) were used instead of Aromatic heterocyclic derivative (H1) as the second hole-transporting material. Table 2 shows the results.

### Comparative Examples 2-1 and 2-2

Organic EL devices were produced and evaluated in the same manner as in Example 2-1, except that Comparative compounds 1 and 2 were used instead of Aromatic heterocyclic derivative (H1) as the second hole-transporting material. Table 2 shows the results.

**Table 2**

| | | Second Hole-transporting material | Measurement results | | |
|---|---|---|---|---|---|
| | | | Luminous efficiency (cd/A) | Driving voltage (V) | Half life (hour) |
| | | | @1mA/cm² | @1mA/cm² | |
| Example | 2-1 | H1 | 38 | 4.8 | 100 |
| | 2-2 | H2 | 35 | 5.2 | 120 |
| | 2-3 | H3 | 37 | 4.9 | 110 |
| Com. Ex. | 2-1 | Comparative compound 1 | 20 | 4.5 | 20 |
| | 2-2 | Comparative compound 2 | 26 | 4.6 | 30 |

As shown in Table 2, the organic EL devices using in the hole-transporting layer the aromatic heterocyclic derivatives according to the invention could have a higher luminous efficiency and longer life compared with the organic EL devices using the comparative compounds.

### INDUSTRIAL APPLICABILITY

The aromatic heterocyclic derivative of the invention can be used in luminescent and phosphorescent organic EL devices.
The organic EL device of the invention can be used as a planar emitting body such as a flat panel display of a wall-hanging television, a copier, a printer or backlight of a liquid crystal display, light sources for instruments, a display panel, a navigation light, and the like.

Although only some exemplary embodiments and/or examples of this invention have been described in detail above, those skilled in the art will readily appreciated that many modifications are possible in the exemplary embodiments and/or examples without materially departing from the novel teachings and advantages of this invention. Accordingly, all such modifications are intended to be included within the scope of this invention.
The contents of the above-described documents are herein incorporated by reference in its entirety.

## Claims

1. An aromatic heterocyclic derivative represented by the following formula (1): 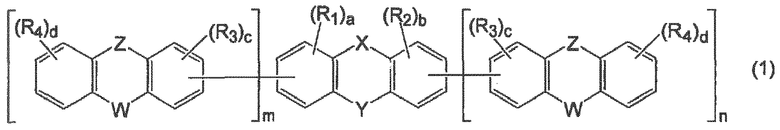 wherein in the formula (1),
X is CRaRb or SiRaRb,
Y and W are independently NRc, an oxygen atom or a sulfur atom,
Z is a single bond, CRaRb or SiRaRb,
Ra, Rb and Rc are independently a hydrogen atom, a substituted or unsubstituted alkyl group having 1 to 15 carbon atoms, a substituted or unsubstituted cycloalkyl group having 3 to 15 ring carbon atoms, a substituted or unsubstituted aralkyl group having 7 to 25 carbon atoms, a substituted or unsubstituted fluoroalkyl group having 1 to 15 carbon atoms, a substituted or unsubstituted aryl group having 6 to 24 ring carbon atoms or a substituted or unsubstituted heteroaryl group having 5 to 24 ring atoms,
R₁, R₂, R₃ and R₄ are independently a substituted or unsubstituted alkyl group having 1 to 15 carbon atoms, a substituted or unsubstituted cycloalkyl group having 3 to 15 ring carbon atoms, a substituted or unsubstituted alkoxy group having 1 to 15 carbon atoms, a substituted or unsubstituted aralkyl group having 7 to 25 carbon atoms, a substituted or unsubstituted fluoroalkyl group having 1 to 15 carbon atoms, a substituted or unsubstituted fluoroalkoxy group having 1 to 15 carbon atoms, a substituted or unsubstituted silyl group, a substituted or unsubstituted aryl group having 6 to 24 ring carbon atoms, a substituted or unsubstituted heteroaryl group having 5 to 24 ring atoms, a substituted or unsubstituted amino group, a substituted or unsubstituted aryloxy group having 6 to 24 ring carbon atoms, a substituted or unsubsituted heteroaryloxy group having 5 to 24 ring atoms, a halogen atom or a cyano group,
a, b and d are independently an integer of 0 to 4,
c is an integer of 0 to 3,
when a to d independently are an integer of 2 or more, two or more of each of R₁s to R₄s may be bonded to each other to form a saturated or unsaturated divalent group,
m and n are independently an integer of 0 to 4, and
the substituent of each of the "a substituted or unsubstituted" group is a group selected from the group consisting of an alkyl group having 1 to 15 carbon atoms, a cycloalkyl group having 3 to 15 ring carbon atoms, an alkoxy group having 1 to 15 carbon atoms, an aralkyl group having 7 to 25 carbon atoms, a fluoroalkyl group having 1 to 15 carbon atoms, a fluoroalkoxy group having 1 to 15 carbon atoms, a silyl group, an alkylsilyl group having 3 to 30 carbon atoms, an arylsilyl group having 8 to 30 carbon atoms, an aryl group having 6 to 24 ring carbon atoms, a heteroaryl group having 5 to 24 ring atoms, an aryloxy group having 6 to 24 ring carbon atoms, a heteroaryloxy group having 5 to 24 ring atoms, a halogen atom and a cyano group,
provided that the substituent is not a 9,10-dihydroacridinyl group.

2. The aromatic heterocyclic derivative according to claim1 represented by the following formula (2): 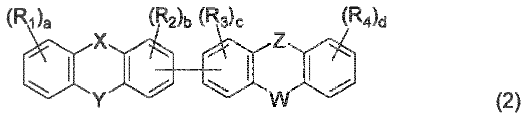 wherein in the formula (2),
X is CRaRb or SiRaRb,
Y and W are independently NRc, an oxygen atom or a sulfur atom,
Z is a single bond, CRaRb or SiRaRb,
Ra, Rb and Rc are independently a hydrogen atom, a substituted or unsubstituted alkyl group having 1 to 15 carbon atoms, a substituted or unsubstituted cycloalkyl group having 3 to 15 ring carbon atoms, a substituted or unsubstituted aralkyl group having 7 to 25 carbon atoms, a substituted or unsubstituted fluoroalkyl group having 1 to 15 carbon atoms, a substituted or unsubstituted aryl group having 6 to 24 ring carbon atoms or a substituted or unsubstituted heteroaryl group having 5 to 24 ring atoms,
R₁, R₂, R₃ and R₄ are independently a substituted or unsubstituted alkyl group having 1 to 15 carbon atoms, a substituted or unsubstituted cycloalkyl group having 3 to 15 ring carbon atoms, a substituted or unsubstituted alkoxy group having 1 to 15 carbon atoms, a substituted or unsubstituted aralkyl group having 7 to 25 carbon atoms, a substituted or unsubstituted fluoroalkyl group having 1 to 15 carbon atoms, a substituted or unsubstituted fluoroalkoxy group having 1 to 15 carbon atoms, a substituted or unsubstituted silyl group, a substituted or unsubstituted aryl group having 6 to 24 ring carbon atoms, a substituted or unsubstituted heteroaryl group having 5 to 24 ring atoms, a substituted or unsubstituted amino group, a substituted or unsubstituted aryloxy group having 6 to 24 ring carbon atoms, a substituted or unsubsituted heteroaryloxy group having 5 to 24 ring atoms, a halogen atom or a cyano group,
a and d are independently an integer of 0 to 4,
b and c are an integer of 0 to 3,
when a to d are independently an integer of 2 or more, two or more of each of R₁s to R₄s may be bonded to each other to form a saturated or unsaturated divalent group, and
the substituent of each of the "a substituted or unsubstituted" group is a group selected from the group consisting of an alkyl group having 1 to 15 carbon atoms, a cycloalkyl group having 3 to 15 ring carbon atoms, an alkoxy group having 1 to 15 carbon atoms, an aralkyl group having 7 to 25 carbon atoms, a fluoroalkyl group having 1 to 15 carbon atoms, a fluoroalkoxy group having 1 to 15 carbon atoms, a silyl group, an alkylsilyl group having 3 to 30 carbon atoms, an arylsilyl group having 8 to 30 carbon atoms, an aryl group having 6 to 24 ring carbon atoms, a heteroaryl group having 5 to 24 ring atoms, an aryloxy group having 6 to 24 ring carbon atoms, a heteroaryloxy group having 5 to 24 ring atoms, a halogen atom and a cyano group,
provided that the substituent is not a 9,10-dihydroacridinyl group.

3. The aromatic heterocyclic derivative according to claim 2 represented by the following formula (3): 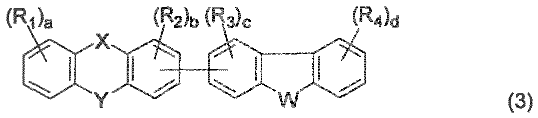 wherein in the formula (3),
X is CRaRb or SiRaRb,
Y and W are independently NRc, an oxygen atom or a sulfur atom,
Ra, Rb and Rc are independently a hydrogen atom, a substituted or unsubstituted alkyl group having 1 to 15 carbon atoms, a substituted or unsubstituted cycloalkyl group having 3 to 15 ring carbon atoms, a substituted or unsubstituted aralkyl group having 7 to 25 carbon atoms, a substituted or unsubstituted fluoroalkyl group having 1 to 15 carbon atoms, a substituted or unsubstituted aryl group having 6 to 24 ring carbon atoms or a substituted or unsubstituted heteroaryl group having 5 to 24 ring atoms,
R₁, R₂, R₃ and R₄ are independently a substituted or unsubstituted alkyl group having 1 to 15 carbon atoms, a substituted or unsubstituted cycloalkyl group having 3 to 15 ring carbon atoms, a substituted or unsubstituted alkoxy group having 1 to 15 carbon atoms, a substituted or unsubstituted aralkyl group having 7 to 25 carbon atoms, a substituted or unsubstituted fluoroalkyl group having 1 to 15 carbon atoms, a substituted or unsubstituted fluoroalkoxy group having 1 to 15 carbon atoms, a substituted or unsubstituted silyl group, a substituted or unsubstituted aryl group having 6 to 24 ring carbon atoms, a substituted or unsubstituted heteroaryl group having 5 to 24 ring atoms, a substituted or unsubstituted amino group, a substituted or unsubstituted aryloxy group having 6 to 24 ring carbon atoms, a substituted or unsubsituted heteroaryloxy group having 5 to 24 ring atoms, a halogen atom or a cyano group,
a and d are independently an integer of 0 to 4,
b and c are an integer of 0 to 3,
when a to d are independently an integer of 2 or more, two or more of each of R₁s to R₄s may be bonded to each other to form a saturated or unsaturated divalent group, and
the substituent of each of the "a substituted or unsubstituted" group is a group selected from the group consisting of an alkyl group having 1 to 15 carbon atoms, a cycloalkyl group having 3 to 15 ring carbon atoms, an alkoxy group having 1 to 15 carbon atoms, an aralkyl group having 7 to 25 carbon atoms, a fluoroalkyl group having 1 to 15 carbon atoms, a fluoroalkoxy group having 1 to 15 carbon atoms, a silyl group, an alkylsilyl group having 3 to 30 carbon atoms, an arylsilyl group having 8 to 30 carbon atoms, an aryl group having 6 to 24 ring carbon atoms, a heteroaryl group having 5 to 24 ring atoms, an aryloxy group having 6 to 24 ring carbon atoms, a heteroaryloxy group having 5 to 24 ring carbon atoms, a halogen atom and a cyano group,
provided that the substituent is not a 9,10-dihydroacridinyl group.

4. The aromatic heterocyclic derivative according to claim 3 represented by the following formula (4): 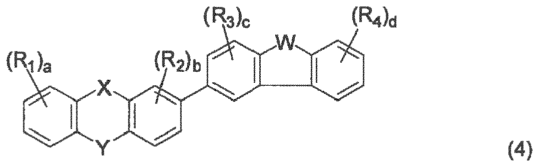 wherein in the formula (4),
X is CRaRb or SiRaRb,
Y and W are independently NRc, an oxygen atom or a sulfur atom,
Ra, Rb and Rc are independently a hydrogen atom, a substituted or unsubstituted alkyl group having 1 to 15 carbon atoms, a substituted or unsubstituted cycloalkyl group having 3 to 15 ring carbon atoms, a substituted or unsubstituted aralkyl group having 7 to 25 carbon atoms, a substituted or unsubstituted fluoroalkyl group having 1 to 15 carbon atoms, a substituted or unsubstituted aryl group having 6 to 24 ring carbon atoms or a substituted or unsubstituted heteroaryl group having 5 to 24 ring atoms,
R₁, R₂, R₃ and R₄ are independently a substituted or unsubstituted alkyl group having 1 to 15 carbon atoms, a substituted or unsubstituted cycloalkyl group having 3 to 15 ring carbon atoms, a substituted or unsubstituted alkoxy group having 1 to 15 carbon atoms, a substituted or unsubstituted aralkyl group having 7 to 25 carbon atoms, a substituted or unsubstituted fluoroalkyl group having 1 to 15 carbon atoms, a substituted or unsubstituted fluoroalkoxy group having 1 to 15 carbon atoms, a substituted or unsubstituted silyl group, a substituted or unsubstituted aryl group having 6 to 24 ring carbon atoms, a substituted or unsubstituted heteroaryl group having 5 to 24 ring atoms, a substituted or unsubstituted amino group, a substituted or unsubstituted aryloxy group having 6 to 24 ring carbon atoms, a substituted or unsubsituted heteroaryloxy group having 5 to 24 ring atoms, a halogen atom or a cyano group,
a and d are independently an integer of 0 to 4,
b and c are an integer of 0 to 3,
when a to d are independently an integer of 2 or more, two or more of each of R₁s to R₄s may be bonded to each other to form a saturated or unsaturated divalent group, and
the substituent of each of the "a substituted or unsubstituted" group is a group selected from the group consisting of an alkyl group having 1 to 15 carbon atoms, a cycloalkyl group having 3 to 15 ring carbon atoms, an alkoxy group having 1 to 15 carbon atoms, an aralkyl group having 7 to 25 carbon atoms, a fluoroalkyl group having 1 to 15 carbon atoms, a fluoroalkoxy group having 1 to 15 carbon atoms, a silyl group, an alkylsilyl group having 3 to 30 carbon atoms, an arylsilyl group having 8 to 30 carbon atoms, an aryl group having 6 to 24 ring carbon atoms, a heteroaryl group having 5 to 24 ring atoms, an aryloxy group having 6 to 24 ring carbon atoms, a heteroaryloxy group having 5 to 24 ring atoms, a halogen atom or a cyano group,
provided that the substituent is not a 9,10-dihydroacridinyl group.

5. The aromatic heterocyclic derivative according to any of claims 1 to 4, wherein X is CRaRb.

6. The aromatic heterocyclic derivative according to any of claims 1 to 5, wherein Y is NRc.

7. The aromatic heterocyclic derivative according to any of claims 1 to 6, which is a material for an organic electroluminescence device.

8. The aromatic heterocyclic derivative according to claim 7, which is a hole-transporting material for an organic electroluminescence device.

9. An organic electroluminescence device comprising:
a cathode, an anode, and one or more organic thin film layers therebetween,
wherein one or more layer of the organic thin film layers comprises the aromatic heterocyclic derivative according to any of 1 to 8 claims.

10. The organic electroluminescence device according to claim 9, wherein the organic thin film layers comprise a hole-transporting layer and/or a hole-injecting layer, and the hole-transporting layer and/or the hole-injecting layer comprises the aromatic heterocyclic derivative.

11. The organic electroluminescence device according to claim 10, wherein a layer comprising a compound represented by the following formula (10) is in contact with to the hole-transporting layer and/or the hole-injecting layer: 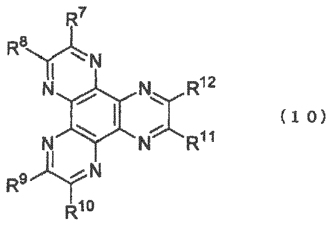 wherein in the formula (10),
R⁷ to R¹² are independently a cyano group, -CONH₂, a carboxy group or -COOR¹³ wherein R¹³ is an alkyl group having 1 to 20 carbon atoms, or -CO-O-CO- formed by bonding of R⁷ and R⁸, R⁹ and R¹⁰, or R¹¹ and R¹².

12. The organic electroluminescence device according to any of claims 9 to 11, wherein the organic thin film layers comprise an emitting layer, and the emitting layer comprises a phosphorescent material.

13. The organic electroluminescence device according to claim 12, wherein the phosphorescent material is an ortho-metalated complex of iridium (Ir), osmium (Os) or platinum (Pt).

14. The organic electroluminescence device according to any one of claims 9 to 13, wherein the organic thin film layers comprise an electron-transporting layer, and the electron-transporting layer comprises the nitrogen-containing heterocyclic derivative represented by any of the following formulas: 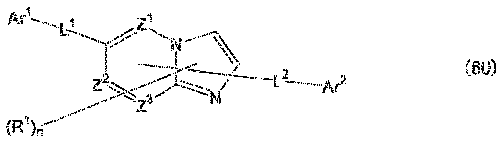 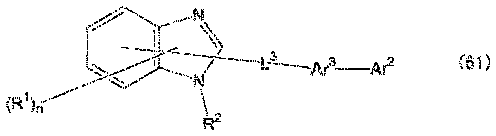 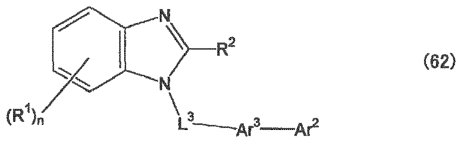 wherein, Z¹, Z² and Z³ are independently a nitrogen atom, a carbon atom or C-H,
R¹ and R² are independently a substituted or unsubstituted aryl group having 6 to 50 ring carbon atoms, a substituted or unsubstituted heteroaryl group having 3 to 50 ring atoms, an alkyl group having 1 to 20 carbon atoms, an alkyl group having 1 to 20 carbon atoms which is substituted with a halogen atom or an alkoxy group having 1 to 20 carbon atoms,
n is an integer of 0 to 5,
when n is an integer of 2 or more, plural R¹s may be the same or different, or adjacent two R¹s may be bonded to each other to form a substituted or unsubstituted aromatic hydrocarbon ring,
Ar¹ is a substituted or unsubstituted aryl group having 6 to 50 ring carbon atoms or a substituted or unsubstituted heteroaryl group having 3 to 50 ring atoms, and
Ar² is a halogen atom, an alkyl group having 1 to 20 carbon atoms, an alkyl group having 1 to 20 carbon atoms which is substituted with an halogen atom, an alkoxy group having 1 to 20 carbon atoms, a substituted or unsubstituted aryl group having 6 to 50 ring carbon atoms or a substituted or unsubstituted heteroaryl group having 3 to 50 ring atoms,
provided that one of Ar¹ and Ar² is a substituted or unsubstituted fused aromatic hydrocarbon ring group having 10 to 50 ring carbon atoms or a substituted or unsubstituted fused aromatic heterocyclic group having 9 to 50 ring atoms,
Ar³ is a substituted or unsubstituted arylene group having 6 to 50 ring carbon atoms or a substituted or unsubstituted heteroarylene group having 3 to 50 ring atoms, and
L¹, L² and L³ are independently a single bond, a substituted or unsubstituted arylene group having 6 to 50 ring carbon atoms or a substituted or unsubstituted hetero fused ring group having 9 to 50 ring atoms.
